# EUROPEAN PATENT SPECIFICATION

(11) **EP 3 787 735 B1**
(45) Date of publication and mention of the grant of the patent: **28.01.2026**
(21) Application number: 19721274.9
(22) Date of filing: 30.04.2019
(51) Int. Cl.: A61N 1/05, A61N 1/36, A61N 1/372, A61B 5/00, A61B 5/026, A61B 5/291, A61B 5/0205

(54) **NEURAL INTERFACE SYSTEM**
NEURALSCHNITTSTELLENSYSTEM
SYSTÈME D'INTERFACE NEURONALE

(30) Priority: 01.05.2018 US 201862665486 P
(43) Date of publication of application: 10.03.2021
(73) Proprietor: Wyss Center for Bio and Neuro Engineering, 1202 Geneva (CH)
(72) Inventor: DONOGHUE, John P., PROVIDENCE, RI 02906 (US); KOUVAS, Georgios, 1202 GENEVA (CH); SOBOLEWSKI, Aleksander, 1205 GENEVE (CH); ZIMMERMANN, Jonas, 1260 NYON (CH); VLACHOS, Ioannis, 01280 PREVESSIN-MOENS (FR); BAUD, Maxime, 3007 BERN (CH); FLAHERTY, J. Christopher, AUBURNDALE, FL 33823 (US)
(74) Representative: HGF
(86) International application number: PCT/EP2019/061129
(87) International publication number: WO 2019/211314

(56) References cited:
- WO-A1-2017/072769
- US-A1- 2006 293 723
- US-A1- 2010 145 176
- US-A1- 2016 206 236
- US-A1- 2017 035 316
- US-A1- 2017 252 568
- RANGANATHA SITARAM ET AL: "Closed-loop brain training: the science of neurofeedback", NATURE REVIEWS. NEUROSCIENCE, vol. 18, no. 2, February 2017 (2017-02-01), GB, pages 86 - 100, XP055604710, ISSN: 1471-003X, DOI: 10.1038/nrn.2016.164

## Description

### Field of the Invention

The present invention relates generally to systems for diagnosing and/or treating a patient disease or disorder, and in particular to neural interface systems that include an implanted sensor assembly.

### Background

Neurology, neurorehabilitation, psychiatry and sleep medicine are fields of medicine that provide care for disorders characterized by chronic cerebral dysfunction. Yet, no apparatus is currently capable of gauging global cerebral function through continuous multimodal physiological recordings over timescales of months to years. Today, monitoring and intervention in brain activity is generally accomplished by devices placed on the scalp or inside the skull. The former is problematic even when monitoring during periods of two to three weeks because it requires daily re-pasting of the electrodes, it is uncomfortable and cumbersome for patients. The latter, which can enable neurostimulation and yields high resolution stable EEG recordings, requires an extensive surgery (craniotomy) and direct contact between electrodes and the brain, which comes with a small yet serious surgical risk. For these and other reasons, chronic intracranial device applications with brain recording capabilities remain a palliative option, when all other possibilities have been exhausted. As intracranial recordings are necessarily focal, there is to date no existing solution for chronic monitoring of global brain activity.

US2017/035316 discloses and implantable body configured for implantation in a subgaleal extracranial position, the implantable body including a first electrode array including a first elongated body comprising first and second electrode contacts separated from one another by a distance selected to facilitate the detection of brain electrical activity and a unit coupled to the first electrode array. The unit includes a processor analyzing the detected brain electrical activity to determine whether an epileptic event has occurred and generating epileptic event data based on this determination and a transceiver controlled by the processor to wirelessly transmit epileptic event data to and from a remote computing device.

US2006/293723 discloses a system and method for applying electrical stimulation or drug infusion to nervous tissue of a patient to treat epilepsy, movement disorders, and other indications uses at least one implantable system control unit (SCU), including an implantable signal/pulse generator (IPG) and one or more electrodes. The IPG is implanted in the mastoid area of the skull and communicates with at least one external appliance, such as a Behind-the-Ear (BTE) unit. In a preferred embodiment, the system is capable of open- and closed-loop operation. In closed-loop operation, at least one SCU includes a sensor, and the sensed condition is used to adjust stimulation parameters.

WO2017/072769 discloses a system that includes a parenchymal electrode, configured to be implanted in brain parenchyma of a subject identified as at risk of or suffering from a disease; and a cerebrospinal fluid (CSF) electrode, configured to be implanted in a CSF-filled space of a brain of the subject, the CSF-filled space selected from the group consisting of: a ventricular system and a subarachnoid space. Control circuitry is configured to drive the parenchymal electrode and the CSF electrode to clear a substance from the brain parenchyma into the CSF-filled space of the brain.

US2010/145176 discloses a seizure advisory system that includes an electrode array having fewer than 32 electrodes in a predetermined dispersed radial pattern, adapted to be implanted through a single opening in the skull, and to be deployed in the predetermined dispersed radial pattern to detect a brain activity signal. The system also includes a communication assembly and an external assembly.

US2016/206236 discloses systems and methods for managing epilepsy. In one embodiment, a method characterizes a patient's propensity for a future epileptic seizure and communicates to the patient and/or a health care provider a therapy recommendation. The therapy recommendation is typically a function of the patient's propensity for the future epileptic seizure.

Accordingly, there is a need for improved systems for diagnosing, prognosing, and treating brain and other patient disorders.

### Summary

The present invention is defined by the appended claims.

Aspects, embodiments, examples and methods described hereinafter are only of exemplary nature and presented for better understanding the present invention.

Exemplary systems, devices and methods described herein can be directed to diagnosing, prognosing, and/or treating a patient brain disorder.

According to an aspect of the present disclosure, a neural interface system for a patient comprises an implantable sensor device comprising: an implantable lead assembly for implantation above the skull and below the skin of the patient, and for recording physiologic parameter information of the patient; and an implantable transmitter for receiving the physiologic parameter information from the implantable lead assembly and for transmitting patient data that is based on the physiologic parameter information. The system further comprises an external processing device for receiving the patient data from the implantable transmitter.

In some embodiments, the system is configured to continuously provide information representing the recorded physiologic parameter information. The recorded physiologic parameter information can comprise neural activity of the patient.

In some embodiments, the system is configured to continuously provide a brain-machine interface function.

In some embodiments, the system is configured to allow the patient to report a neurological event. The neurological event can comprise a seizure and/or other epileptic event.

In some embodiments, the system is configured to diagnose, prognose, and/or treat a medical condition selected from the group consisting of: brain condition; neurological condition; epilepsy; coma; psychiatric disorder; mood disorder; obsessive compulsive disorder; an attention deficit disorder; Tourette's syndrome; a neurodegenerative disease; a movement disorder; essential tremor; Parkinson's disease; a tic disorder; sleep disorder; pain; neuropathic pain; stroke; paralysis; tinnitus; dyslexia; a speech production disorder, such as aphasia; a memory disorder, such as amnesia; chronic fatigue syndrome; migraine headaches; multiple sclerosis; a chronic demyelinating disorder; and combinations thereof.

In some embodiments, the system is configured to function as a brain-machine interface.

In some embodiments, the system is configured to provide a warning of the occurrence of a neurological event. The neurological event can comprise a seizure and/or an upcoming seizure.

In some embodiments, the system is configured predict the occurrence of a neurological event. The predicted neurological event can comprise a present seizure and/or future seizure.

In some embodiments, the system is configured to determine a risk of occurrence of a neurological event. The neurological event can comprise a present seizure and/or future seizure. The risk of occurrence can be compared to a threshold, and the patient can be alerted if the risk of occurrence exceeds the threshold.

In some embodiments, the system is configured to provide dynamic informed therapy. The system can be configured to cause an adjustment of a drug and/or other agent being delivered to the patient. The system can further comprise an agent delivery pump, and the system can adjust the drug and/or other agent delivered by the pump. The system can be configured to provide information related to a suggested adjustment of a drug and/or other agent for delivery to the patient.

In some embodiments, the system is configured to perform electrical impedance tomography. The system can be configured to monitor seizures of the patient.

In some embodiments, the system is configured to perform temporal interference electrical stimulation. The implantable lead assembly can comprise at least three electrodes configured in at least two pairs, and the implantable sensor device can be configured to generate an electric field in tissue via each pair of electrodes. The implantable sensor device can generate a first field using a first set of electrodes and a second field using a second set of electrodes, and the first field and second field can be generated using a first drive signal at a first frequency and a second drive signal at a second frequency, and the first frequency and the second frequency can comprise a difference of at least 2 Hz. The system can be configured to deliver stimulation to a sulcus of the cortex of the brain. The system can be configured to deliver stimulation to tissue at least 10mm below the surface of the brain. The system can be configured to deliver stimulation to tissue at least 20mm below the surface of the brain. The system can be configured to deliver stimulation to tissue at least 40mm below the surface of the brain.

In some embodiments, the system is configured to provide feedback to the patient. The feedback provided can comprise neurofeedback.

In some embodiments, the system is configured to receive feedback from the patient.

In some embodiments, the system is configured to process information to perform a medical procedure, and the information comprises information selected from the group consisting of: neuronal activity; brain activity; activity in the brain cortex; activity in the deep brain; muscle activity; action potential activity; glucose levels; blood pressure; blood gas levels; pH of a body fluid; skin conductance; electrodermal activity; tissue temperature; body fluid temperature; heart activity; respiration activity; and combinations thereof.

In some embodiments, at least a portion of the implantable sensor device is biodegradable. The biodegradable portion can comprise at least a portion of a component selected from the group consisting of: a lead or other conduit of the implantable lead assembly; an electrode of the implantable lead; a shaft of the implantable lead; a stimulation element; and combinations thereof. The biodegradable portion can comprise a material selected from the group consisting of: a biodegradable metal; magnesium; a biodegradable plastic; a biodegradable polymer; a conductive biodegradable polymer; polycaprolactone; Pedot-PSS; a biodegradable polyester; and combinations thereof. The biodegradation can comprise energy assisted biodegradation. The implantable sensor device can comprise an energy delivery assembly configured to deliver the biodegradation energy. The system can further comprise an agent configured to be delivered into the patient to cause the biodegradation.

In some embodiments, the implantable sensor device is configured to deliver stimulation to the patient. The implantable lead assembly can deliver the stimulation to the patient. The implantable lead assembly can comprise at least one electrode that delivers the stimulation to the patient. The system can further comprise at least one stimulation element that delivers the stimulation to the patient. The at least one stimulation element can be configured to be positioned on the skull below the patient's skin. The at least one stimulation element can be configured to be positioned in the brain of the patient. The at least one stimulation element can be configured to deliver stimulation in a form selected from the group consisting of: electrical energy; magnetic energy; electro-magnetic energy; light energy; chemical energy; thermal energy; heat energy; cooling energy; sound energy; subsonic energy; ultrasound energy; mechanical energy; an agent; and combinations thereof. The at least one stimulation element can be configured to stimulate the vagal nerve of the patient. The at least one stimulation element can be configured to stimulate the heart of the patient. The at least one stimulation element can be configured to pace and/or defibrillate the heart. The at least one stimulation element can be configured to prevent sudden unexpected death in epilepsy. The system can be configured to deliver stimulation directly and/or indirectly to the cortex of the brain, and the system can be further configured to assess cortical excitability. The stimulation can be delivered by: one or more electrodes of the implantable lead assembly; an intracerebral electrode; and combinations thereof. The implantable lead assembly can comprise at least three electrodes, and a first electrode can be configured as a focal stimulation electrode, and the remaining electrodes can be configured as return electrodes. The system can be configured to deliver trains of single-pulse electrical stimulation and to record the magnitude of the evoked response. The system can be further configured to determine a state of epileptic disease of the patient. The system can be configured to determine susceptibility to neurofeedback and/or neuromodulation therapy.

In some embodiments, the implantable lead assembly is attachable to the implantable transmitter.

In some embodiments, the implantable lead assembly is pre-attached to the implantable transmitter.

In some embodiments, the implantable lead assembly comprises multiple electrodes. The multiple electrodes can comprise at least six electrodes. The multiple electrodes can comprise two or more tubular electrodes. The implantable lead assembly can further comprise at least one lead that includes the two or more tubular electrodes, and the at least one lead can comprise a width of no more than 2.5mm, no more than 2.0mm, and/or no more than 1.5mm. The multiple electrodes comprise two or more facet electrodes that span less than 180 degrees of a circumferential segment, and at least one of the two or more facet electrodes can be oriented toward the skull after implantation. The implantable lead assembly can further comprise at least one shaft, and the multiple electrodes can comprise two or more facet electrodes that can be positioned around a circumference of the at least one shaft. The two or more facet electrodes can be individually selectable. The two or more facet electrodes can comprise a first facet electrode for recording the physiologic parameters and a second facet electrode used for noise suppression. The second facet electrode can be configured to be oriented away from the patient's skull after implantation. The multiple electrodes can comprise at least one concentric ring electrode surrounding a central electrode. The at least one concentric ring electrode can comprise a tripolar concentric electrode. The at least one concentric ring electrode can comprise between two and ten concentric ring electrodes. The at least one concentric ring electrode can comprise between two and five concentric ring electrodes. The concentric ring electrodes can each comprise an outer ring with a diameter of at least 5mm. The concentric ring electrodes can each comprise an outer ring with a diameter of at least 10mm. The implantable lead assembly can further comprise at least one lead with a diameter of no more than 12mm and/or no more than 10mm.

In some embodiments, the implantable lead assembly comprises at least one intra-bone skull electrode comprising a shaft with a proximal end and a distal end and an electrode positioned on and/or in the shaft. The skull electrode can be positioned on the distal end of the shaft. The skull electrode can further comprise a cap positioned on the proximal end of the shaft. The shaft can comprise threads configured to frictionally engage bone. The implantable lead assembly can further comprise at least one lead, and the shaft can be configured to pass through and electrically connect with the at least one lead. The skull electrode can be configured to be flush with the inner table of the skull after implantation. The skull electrode can be configured to be positioned at a location above the inner table of the skull after implantation. The skull electrode can be configured to extend beneath the inner table of the skull after implantation. The electrode can be configured to contact the dura without penetrating the dura. The system can further comprise a tool for rotating the skull electrode such that it engages the skull. The tool can be configured to access the skull electrode via an opening in the skin above the location in which the skull electrode can be inserted into the skull. The tool can be configured to access the skull electrode via an incision in the skin offset from location in which the skull electrode can be inserted into the skull. The implantable lead assembly can be configured to pass through the incision.

In some embodiments, the implantable lead assembly comprises at least one electrode comprising a shielded portion.

In some embodiments, the implantable lead assembly comprises at least one shaft configured to position at least one sensor in brain tissue. The at least one sensor can comprise at least one electrode. The system can further comprise an inserter tool configured to apply a force to the at least one shaft to insert the at least one shaft into brain tissue. The system can further comprise at least one guide tool configured to engage the skull and can guide the at least one shaft into brain tissue. The implantable lead assembly can further comprise a connector configured to electrically connect the at least one sensor to an electrical conduit of the implantable lead assembly. The implantable lead assembly can comprise a hub configured to electrically connect the at least one sensor to the implantable sensor device.

In some embodiments, the implantable lead assembly comprises multiple leads, and each lead comprises at least one electrode. One or more leads of the multiple leads can comprise a material selected from the group consisting of: silicone; a polymer; PDMS; polycaprolactone; a biodegradable material; and combinations thereof. The multiple leads can comprise at least two leads. The multiple leads can comprise at least three leads. The multiple leads can comprise at least four leads. The multiple leads can comprise at least five leads. The multiple leads can comprise at least six leads. The multiple leads can comprise at least eight leads. The multiple leads can comprise at least ten leads. The multiple leads can be configured to be implanted beneath the skin and on top of the skull. At least a portion of a lead can be configured to be implanted under a temporal muscle. The portion of the lead under the temporal muscle can comprise at least one sensor. The at least one sensor can comprise an electrode including a shielded portion, and the shielded portion can be configured to be oriented toward the temporal muscle. The multiple leads can be configured to be implanted in a star shaped geometry. The multiple leads can be configured to be tunneled under the skin. The multiple leads can be configured to be folded into a single tube geometry. Each lead can comprise a width less than or equal to 3.5mm. Each lead can comprise a length of approximately 10cm. Each lead can comprise a length of at least 5cm. Each lead can comprise a length of no more than 15cm. Each lead can comprise between three and ten electrodes. Each lead can comprise between four and five electrodes. Each lead can comprise one or more axial reinforcing elements. Each axial reinforcing element can be configured to withstand stretching and/or twisting of the associated lead. Each axial reinforcing element can be configured to withstand forces encountered during implantation of each lead. The axial reinforcing elements can be configured to withstand forces encountered during explantation of each lead. The axial reinforcing elements can be configured to withstand forces encountered by each lead while implanted. Each axial reinforcing element can comprise a reinforcing filament. The reinforcing filament can comprise suture material. The reinforcing filament can comprise a loop portion that exits the distal end of the associated lead. Each axial reinforcing element can comprise a reinforcing mesh. The reinforcing mesh can comprise a plastic mesh. Each lead can comprise a reinforced tip. The system can further comprise a first tunneling tool configured to engage the reinforced tip and tunnel the lead through tissue. The system can further comprise a second tunneling tool configured to create a tunnel in tissue for the first tunneling tool to pass through. The tunneling tool can comprise forceps. The reinforced tip can comprise a reinforcing element. The reinforcing element can comprise reinforcing metal and/or plastic. Each lead can comprise a shaft comprising the reinforced tip, and the reinforced tip can comprise a higher durometer material than the remainder of the shaft. Each lead can comprise a distal end and an attachment element positioned proximate the distal end. The attachment element can comprise an aperture. The attachment element can comprise a magnet. The implantable lead assembly can comprise a central conduit operably attached to the implantable transmitter and to each of the multiple leads. The multiple leads can be arranged in a staggered geometry, and each lead can extend from the central conduit. Each lead can depart from the central conduit with a takeoff angle of at least 5°. Each lead can comprise at least one stabilizing projection. The implantable lead assembly can be configured to be implanted into the patient via a single incision above the skull and a single incision behind the ear. The implantable lead assembly can be configured to be inserted through an incision of no more than 5cm. The implantable lead assembly can be configured to be inserted through an incision of no more than 3cm. The implantable lead assembly can be configured to be inserted through an incision of no more than 2cm. The implantable lead assembly can be configured to be implanted in a geometry that defines a convex hull that can cover at least 10% of the convexity of the cerebral hemisphere of the patient. The defined convex hull can cover at least 50% of the convexity of the cerebral hemisphere of the patient. The defined convex hull can cover at least 75% of the convexity of the cerebral hemisphere of the patient.

In some embodiments, the implantable lead assembly comprises multiple stimulation elements configured to deliver stimulation to the patient. The multiple stimulation elements can comprise one or more stimulation elements selected from the group consisting of: electrode; energy delivery element; electrical energy delivery element; magnetic energy delivery element; light delivery element; sound delivery element; ultrasound delivery element; agent delivery element; and combinations thereof.

In some embodiments, the implantable lead assembly comprises at least one sensor, and the system further comprises a visualizable marker positioned relative to the at least one sensor. The visualizable marker can comprise a marker selected from the group consisting of: radiopaque marker; ultrasound marker; magnetic marker; and combinations thereof. The visualizable marker can comprise an infrared diode. The system can further comprise a tool comprising an imaging device configured to visualize the visualizable marker.

In some embodiments, the physiologic parameter information recorded by the implantable lead assembly represents neural information of the patient.

In some embodiments, the physiologic parameter information recorded by the implantable lead assembly comprises information selected from the group consisting of: neuronal activity; brain activity; activity in the brain cortex; activity in the deep brain; muscle activity; action potential activity; glucose levels; blood pressure; blood gas levels; pH of a body fluid; skin conductance; electrodermal activity; temperature; heart activity; respiration activity; and combinations thereof.

In some embodiments, the physiologic parameter information recorded by the implantable lead assembly comprises a parameter selected from the group consisting of: temperature; skin temperature; heart rate; a measure of motion; a measure of gate; a measure of fatigue; and combinations thereof.

In some embodiments, the physiologic parameter information comprises information recorded by an fNIRS sensor.

In some embodiments, the physiologic parameter information comprises cerebral hemodynamic information.

In some embodiments, the physiologic parameter information comprises information recorded by an electrical impedance tomography sensor.

In some embodiments, the implantable transmitter comprises a wireless transmitter.

In some embodiments, the implantable transmitter is further configured to receive wireless transmissions. The implantable transmitter can be configured to receive wireless transmissions from the external processing device.

In some embodiments, the patient data transmitted by the implantable transmitter comprises the recorded physiologic parameter information.

In some embodiments, the implantable transmitter is configured to process the recorded physiologic parameter information, and the patient data comprises processed physiologic parameter information. The processing of the recorded physiologic parameter information can comprise processing selected from the group consisting of: amplifying; referencing; re-referencing; mathematically processing; digitizing; condensing; compressing; notch filtering; band-pass filtering; scaling; zero-centering; averaging; determining a maximum; determining a minimum; determining a mean; thresholding; transforming; spectrally analyzing; integrating; differentiating; performing signal conditioning; feature extraction; and combinations thereof. The processing of the recorded physiologic parameter information can comprise a feature extraction. The feature extraction can comprise an analysis selected from the group consisting of: temporal analysis; spectral analysis; wavelet analysis; and combinations thereof. The system can be configured to apply a classification regression and/or machine learning algorithm on features extracted from the feature extraction. The processing of the recorded physiologic parameter information can comprise a time series analysis of data recorded by the system. The time series analysis can comprise an analysis selected from the group consisting of: auto-correlation; cross-correlation; stochastic process analysis; chaotic time series analysis; and combinations thereof.

In some embodiments, the implantable transmitter comprises memory storage.

In some embodiments, the implantable transmitter comprises an energy storage assembly. The energy storage assembly can comprise a capacitor and/or a rechargeable battery.

In some embodiments, the implantable transmitter is configured to receive command signals from the external processing device.

In some embodiments, the external processing device is configured to store, condition, and/or process the patient data received from the implantable transmitter.

In some embodiments, the external processing device is configured to perform processing selected from the group consisting of: amplifying; referencing; re-referencing; mathematically processing; digitizing; condensing; compressing; notch filtering; band-pass filtering; scaling; zero-centering; averaging; determining a maximum; determining a minimum; determining a mean; thresholding; transforming; spectrally analyzing; integrating; differentiating; performing signal conditioning; feature extraction; and combinations thereof.

In some embodiments, the external processing device is configured to transmit energy to the implantable transmitter. The external processing device can be configured to transmit the energy using inductive power transfer.

In some embodiments, the external processing device is configured to transmit information to the implantable transmitter.

In some embodiments, the external processing device is configured to be positioned about the patient's head.

In some embodiments, the external processing device comprises a patient input device configured to receive information manually from the patient.

In some embodiments, the system further comprises at least one tool. The implantable lead assembly can comprise at least one lead with an attachment element, and the at least one tool can comprise a tool configured to engage the attachment element to apply a force to the at least one lead. The implantable lead assembly can further comprise at least one intra-bone skull electrode for insertion into bone at an insertion location, and the at least one tool can comprise a right-angle rotation tool configured to rotate the skull electrode via an incision remote from the insertion location. The implantable lead assembly can comprise at least one intra-bone skull electrode for insertion into bone at an insertion location, and the at least one tool can comprise an axial rotation tool configured to rotate the skull electrode via an incision proximate and above the insertion location.

In some embodiments, the system further comprises at least one sensor configured to produce a signal related to a physiologic parameter of the patient. The implantable lead assembly can comprise the at least one sensor. The system can further comprise a second implantable device, and the second implantable device can comprise the at least one sensor. The at least one sensor can be configured to be positioned on the patient's skin to record the signal. The at least one sensor can be configured to be implanted in the patient to record the signal. The at least one sensor can comprise a temperature sensor, a pressure sensor, a heart rate sensor, a motion sensor, and a microphone. The at least one sensor can comprise one or more sensors selected from the group consisting of: electrical activity sensor; electrode; magnetic sensor; light sensor; pressure sensor; force sensor; strain gauge; motion sensor; vibration sensor; accelerometer; gravimetric sensor; pH sensor; temperature sensor; humidity sensor; physiologic sensor; blood pressure sensor; pulse sensor; blood sensor; blood gas sensor; glucose sensor; ion sensor; small molecule sensor; steroid sensor; protein sensor; respiration sensor; and combinations thereof. The implantable lead assembly can comprise the at least one sensor. The at least one sensor can comprise one or more sensors selected from the group consisting of: accelerometer; motion sensor; pressure sensor; gravimetric sensor; magnetic sensor; force sensor; strain gauge; temperature sensor; humidity sensor; light sensor; physiologic sensor; and combinations thereof. The at least one sensor can comprise one or more sensors selected from the group consisting of: electrical activity sensor; electroencephalogram (EEG) sensor; local field potential (LFP)sensor; an electrocorticogram (ECoG) sensor; an electromyogram (EMG) sensor; action potential spike sensor; glucose sensor; pressure sensor; blood gas sensor; blood pressure sensor; pulse sensor; ion sensor; small molecule sensor; steroid sensor; protein sensor; pH sensor; galvanic skin response sensor; electrodermal sensor; temperature sensor; electrocardiogram (ECG or EKG) sensor; respiration sensor; a sphenoidal electrode; a lactate sensor; and combinations thereof. The at least one sensor can comprise a heart rate sensor. The heart rate sensor can comprise a photoplethysmogram sensor. The at least one sensor can comprise an electrodermal sensor. The at least one sensor can comprise a motion sensor. The motion sensor signal can be representative of motor behavior of the patient. The motion sensor signal can be representative of gait, fatigue, and/or falls of the patient. The at least one sensor can comprise a microphone. The microphone can produce a signal representative of commands and/or other verbal information provided by the patient. The at least one sensor can comprise a sphenoidal electrode. The at least one sensor can be positioned on and/or within the external processing device. The at least one sensor can be positioned on and/or within the implantable transmitter.

In some embodiments, the system further comprises at least one intracranial sensor operably connected to the implantable transmitter. The at least one intracranial sensor can comprise ECoG, subdural, and/or depth electrodes. The at least one intracranial sensor can comprise at least one functional near infrared spectroscopy sensor. The at least one functional near infrared spectroscopy sensor can be positioned through and beneath the skull. The at least one functional near infrared spectroscopy sensor can be configured to measure cerebral hemodynamics. The at least one intracranial sensor can comprise at least one foramen ovale electrode.

In some embodiments, the system further comprises a patient input assembly configured to receive feedback comprising patient generated information. The patient input assembly can comprise a patient wearable device. The patient input assembly can be configured to allow the patient to report a neurological event. The neurological event can comprise an epileptic event. The patient input assembly can comprise an accelerometer configured to detect a tap of the patient. The system can be configured to detect dual taps of the patient. The patient input assembly can comprise at least one implanted vibration sensor configured to detect speech and/or other utterances of the patient. The at least one vibration sensor can be configured to detect ictal crying of the patient. The at least one vibration sensor can be configured to engage the skull of the patient. The system can further comprise a feedback assembly configured to provide feedback to the patient.

In some embodiments, the system further comprises a data logging module configured to receive patient information from the external processing device. The patient information can comprise the physiologic parameter information recorded by the implantable sensor device. The patient information can further comprise other physiologic information recorded by the system. The patient information can comprise information related to brain activity of the patient. The patient information can further comprise other physiologic parameter information of the patient. The data logging module can receive the patient information from the external processing device. The system can further comprise at least one controller which receives information from the external processing device, and the data logging module can receive the patient information from the at least one controller. The system can further comprise a computer network, and the data logging module can receive the patient information via the computer network. The computer network can comprise the internet. The computer network can be configured to perform long-term patient information logging. The computer network can be configured to analyze the patient information received from the external processing device. The computer network can be configured to allow analysis of the received patient information by a user. The computer network can be configured to periodically receive the patient information from the external processing device.

In some embodiments, the system further comprises a clinician programmer configured to control the implantable transmitter and/or the external processing device.

In some embodiments, the system further comprises a user interface. The user interface can comprise a display for providing visual information to at least the patient. The user interface can comprise a speaker for providing audible information to at least the patient. At least a portion of the user interface can be positioned on the external processing device.

In some embodiments, the system further comprises a feedback assembly that provides feedback to the patient. The system can be configured to provide continuously available feedback. The system can be configured to monitor brain activity, and the feedback assembly can alert the patient when particular brain activity is detected. The system can be configured to perform multiparametric monitoring of brain activity. The system can further comprise a physiologic sensor configured to record one or more physiologic parameters of the patient, and the system can be configured to assess brain activity and to assess the one or more physiologic parameters recorded by the physiologic sensor, and the system can be configured to provide feedback to the patient based on the assessed brain activity and the physiologic parameters recorded by the physiologic sensor. The feedback assembly can comprise an alert element configured to provide the feedback to the patient. The alert element can comprise an element selected from the group consisting of: visual display; speaker; light; tactile transducer; and combinations thereof. The alert element can be positioned external to the patient. The alert element can be implanted in the patient. The alert element can be positioned within the implantable sensor device. The alert element can be positioned within the implantable transmitter.

In some embodiments, the system further comprises a therapeutic device configured to provide a therapeutic treatment to the patient. The therapeutic device can be configured to receive information from one or more components of the system, and can deliver therapy based on the received information. The system component can comprise the implantable sensor device and/or the external processing device. The therapeutic device can comprise an implantable device. The therapeutic device can comprise a stimulator. The therapeutic device can comprise a drug and/or other agent delivery pump. The therapeutic device can be configured to deliver the drug and/or other agent to an anatomical location selected from the group consisting of: the skin; the mouth or other gastrointestinal location; subcutaneous tissue; a vein; an artery; a muscle; the heart; the brain; a ventricle of the brain; below the dura above the brain; the spine; the epidural space; the intrathecal space; and combinations thereof. The therapeutic device can be configured to deliver a drug selected from the group consisting of: anti-epileptic drug; pain alleviating drug; psychiatric drug; neuropsychopharmacology drug; antidepressant; anesthetic; and combinations thereof. The therapeutic device can comprise a transceiver configured to transfer information to and/or from the implantable sensor device. The therapeutic device can comprise a transceiver configured to transfer information to and/or from the external processing device.

According to another aspect of the present disclosure, an exemplary method of performing a medical procedure on a patient comprises implanting an implantable sensor device into the patient, recording physiologic parameter information, and transmitting the recorded information to an external processing device.

The technology described herein, along with the attributes and attendant advantages thereof, will best be appreciated and understood in view of the following detailed description taken in conjunction with the accompanying drawings in which exemplary embodiments are described by way of example.

### Brief description of the drawings

Fig. 1 illustrates a schematic view of a neural interface system comprising an implantable sensor device and an external processing device.
Fig. 2 illustrates a schematic view of another embodiment of a neural interface system comprising an implantable sensor device and an external processing device.
Figs. 3A-C illustrate top views of three lead assemblies.
Figs. 4A-C illustrate views of a lead assembly comprising a bone penetrating sensor.
**Fig. 4D** illustrates an anatomical view of the bone penetrating sensor of Figs. 4A-Cbeing implanted in a patient.
Figs. 5A-B illustrate a top view of two lead assemblies.
Fig. 6 illustrates a perspective view of a patient's head into which a lead assembly and implantable transmitter have been implanted.
Figs. 7A-C illustrate side sectional views of an inserter tool, a lead, and an inserter tool engaged with the lead, respectively.
Figs. 8A-C illustrate views of leads comprising reinforcing members.
Fig. 9 illustrates a top view of a lead assembly comprising a staggered arrangement of leads.
Fig. 10 illustrates a perspective view of a lead comprising at least one tubular sensor.
Fig. 11 illustrates a perspective view of a lead comprising multiple circumferentially placed sensors.
Figs. 12A-B illustrate sectional anatomical views of a lead being inserted into a patient's brain.

### Detailed description of the drawings

Reference will now be made in detail to the disclosed exemplary embodiments of the technology, examples of which are illustrated in the accompanying drawings. Similar reference numbers may be used to refer to similar components. However, the description is not intended to limit the present disclosure to particular embodiments, and it should be construed as including various modifications, equivalents, and/or alternatives of the embodiments described herein.

The scope of the present invention is defined by the appended claims.

It will be understood that the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") when used herein, specify the presence of stated features, integers, steps, operations, elements, and/or components, but do not preclude the presence or addition of one or more other features, integers, steps, operations, elements, components, and/or groups thereof.

It will be further understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on", "attached", "connected" or "coupled" to another element, it can be directly on or above, or connected or coupled to, the other element, or one or more intervening elements can be present. In contrast, when an element is referred to as being "directly on", "directly attached", "directly connected" or "directly coupled" to another element, there are no intervening elements present. Other words used to describe the relationship between elements should be interpreted in a like fashion (e.g. "between" versus "directly between," "adjacent" versus "directly adjacent," etc.).

It will be further understood that when a first element is referred to as being "in", "on" and/or "within" a second element, the first element can be positioned: within an internal space of the second element, within a portion of the second element (e.g. within a wall of the second element); positioned on an external and/or internal surface of the second element; and combinations thereof.

As used herein, the term "proximate" shall include locations relatively close to, on, in and/or within a referenced component, anatomical location, or other location.

Spatially relative terms, such as "beneath," "below," "lower," "above," "upper" and the like may be used to describe an element and/or feature's relationship to another element(s) and/or feature(s) as, for example, illustrated in the figures. It will be further understood that the spatially relative terms are intended to encompass different orientations of the device in use and/or operation in addition to the orientation depicted in the figures. For example, if the device in a figure is turned over, elements described as "below" and/or "beneath" other elements or features would then be oriented "above" the other elements or features. The device can be otherwise oriented (e.g. rotated 90 degrees or at other orientations) and the spatially relative descriptors used herein interpreted accordingly.

The terms "reduce", "reducing", "reduction" and the like, where used herein, are to include a reduction in a quantity, including a reduction to zero. Reducing the likelihood of an occurrence shall include prevention of the occurrence.

The term "and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

The term "one or more", where used herein can mean one, two, three, four, five, six, seven, eight, nine, ten, or more, up to any number.

The expression "configured (or set) to" used in the present disclosure may be used interchangeably with, for example, the expressions "suitable for", "having the capacity to", "designed to", "adapted to", "made to" and "capable of" according to a situation. The expression "configured (or set) to" does not mean only "specifically designed to" in hardware. Alternatively, in some situations, the expression "a device configured to" may mean that the device "can" operate together with another device or component.

The term "transducer" where used herein is to be taken to include any component or combination of components that receives energy or any input, and produces an output. For example, a transducer can include an electrode that receives electrical energy, and distributes the electrical energy to tissue (e.g. based on the size of the electrode). In some configurations, a transducer converts an electrical signal into any output, such as light (e.g. a transducer comprising a light emitting diode or light bulb), sound (e.g. a transducer comprising a piezo crystal configured to deliver ultrasound energy), pressure, heat energy, cryogenic energy, chemical energy; mechanical energy (e.g. a transducer comprising a motor or a solenoid), magnetic energy, and/or a different electrical signal (e.g. a Bluetooth or other wireless communication element). Alternatively or additionally, a transducer can convert a physical quantity (e.g. variations in a physical quantity) into an electrical signal. A transducer can include any component that delivers energy and/or an agent to tissue, such as a transducer configured to deliver one or more of: electrical energy to tissue (e.g. a transducer comprising one or more electrodes); light energy to tissue (e.g. a transducer comprising a laser, light emitting diode and/or optical component such as a lens or prism); mechanical energy to tissue (e.g. a transducer comprising a tissue manipulating element); sound energy to tissue (e.g. a transducer comprising a piezo crystal); chemical energy; electromagnetic energy; magnetic energy; and combinations thereof.

The term **"medical condition"** where used herein is to be taken to refer to one or more diseases, disorders, and/or other medical condition(s) of a human or other mammalian patient.

The term **"medical procedure"** where used herein is to be taken to refer to a procedure in which a diagnosis is obtained, a prognosis is determined, a treatment is provided, and/or another medical procedure is performed. A "medical procedure" can refer to a single diagnosis, prognosis, and/or treatment, or it can refer to a procedure in which a combination of each, and/or multiples of each, are performed. A medical procedure can be used to assess the condition of human physiology.

The term **"user interface component"** where used herein is to be taken to refer to one or more user input and/or user output components, such as data entry components (touchscreens, buttons, switches, joysticks, and the like), as well as information providing components (e.g. screens, lights, speakers, vibrational transducers, and the like).

Terms defined in the present disclosure are only used for describing specific embodiments of the present disclosure and are not intended to limit the scope of the present disclosure. Terms provided in singular forms are intended to include plural forms as well, unless the context clearly indicates otherwise. All of the terms used herein, including technical or scientific terms, have the same meanings as those generally understood by an ordinary person skilled in the related art, unless otherwise defined herein. Terms defined in a generally used dictionary should be interpreted as having meanings that are the same as or similar to the contextual meanings of the relevant technology and should not be interpreted as having ideal or exaggerated meanings, unless expressly so defined herein. In some cases, terms defined in the present disclosure should not be interpreted to exclude the embodiments of the present disclosure.

Provided herein are neural interface systems and devices, as well as methods for using these components. A neural interface system of the present disclosure includes an implantable sensor device that includes an implantable lead assembly and an implantable transmitter. The implantable lead assembly can be positioned in one or more locations within a patient, such as at a location above the skull and below the skin. The implantable lead assembly includes one or more sensors, such as electrodes, for recording physiologic parameters of the patient (e.g. continuously record physiologic parameters of the patient), such as electrical activity of the patient's brain and/or other neural activity of the patient. The implantable transmitter receives the physiologic parameter information from the implantable lead assembly, and transmits patient data to an external processing device. The transmitted patient data is based on the physiologic parameter information. The system can process the transmitted patient data, such as to provide enhanced diagnosis, prognosis, and/or treatment that can be provided by a treatment component of the system. The system can continuously provide (e.g. to the patient, clinician, and/or other user) information related to the recorded physiologic parameters of the patient. The system can be configured to deliver stimulation to the patient (e.g. electrical, light and/or other stimulation energy and/or a pharmaceutical agent), such as via one or more stimulation elements described herein. The system can include a patient input assembly, such as one or more components configured to receive patient input, also as described herein. The system can receive feedback from a user (e.g. the patient or clinician), and/or provide feedback to a user (e.g. the patient or clinician). In some embodiments, the system provides neurofeedback to the patient, such as during training or while delivering therapy to the patient.

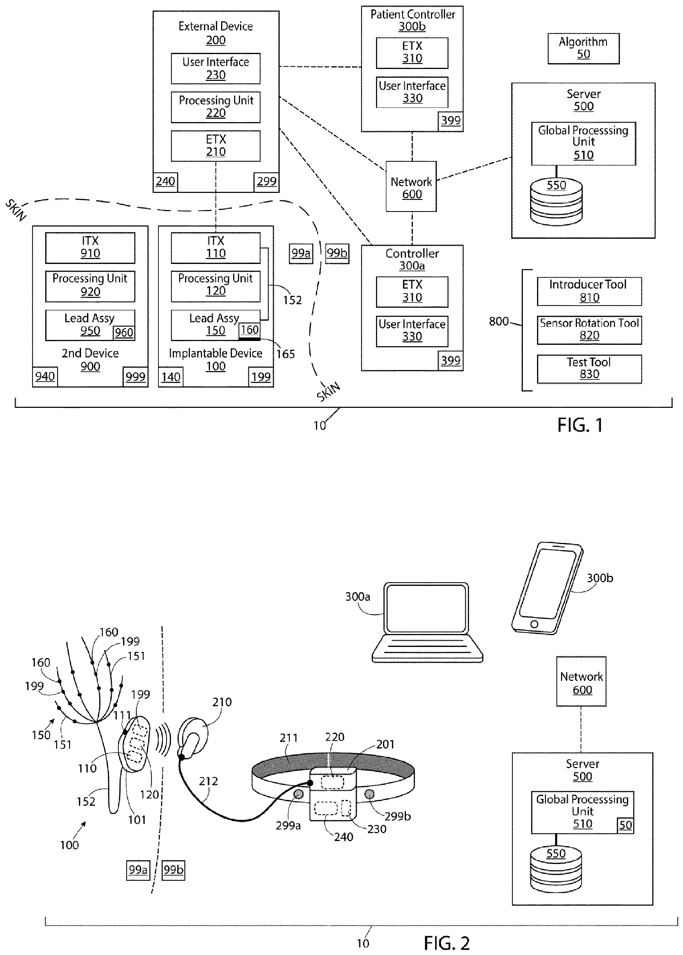

**Referring now to** **Fig. 1****,** a schematic view of a neural interface system including an implantable sensor device and an external processing device is illustrated. **System 10** includes an implantable sensor device, **implantable device 100,** which is configured to be implanted in a patient and to record physiologic parameter information of the patient, such as electrical signals of the patient's brain. System 10 further includes an external processing device, **external device 200,** which is maintained external to the patient's body and receives information ("data" or "information" herein) from implantable device 100. Implantable device 100 includes an implantable transmitter, **ITX 110,** for wirelessly transmitting information, and external device 200 includes a receiver, **ETX 210** for wirelessly receiving information from ITX 110. Implantable device 100 can include **lead assembly 150** which can include one or more electrodes and/or other sensing elements, **sensors 160,** that provide signals related to one or more physiologic parameters of the patient (e.g. electrical activity of the brain and/or other tissue of the patient).

As described hereabove, implantable device 100 can comprise an implantable sensor device that includes lead assembly 150 and ITX 110. In some embodiments, one or more portions of implantable device 100 is constructed and arranged as described herebelow in reference to Figs. 2 - 12. Sensors 160 and other sensors of system 10 can be constructed and arrange to monitor activity (e.g. aggregate electrical activity) of one or more portions of the patient's brain, such as all or portions of the superior aspect of the patient's cerebral cortex. In some embodiments, sensor 160 comprises one or more electrodes configured to record electrical activity and/or deliver electrical energy. In these embodiments, each electrode-based sensor 160 can record electrical activity and/or deliver electrical energy in a monopolar mode (e.g. working with a common electrode such as an electrode-portion of a housing of ITX 110 (e.g. common electrode 111 described herebelow in reference to Fig. 2) or other portion of implantable device 100 configured as a common electrode.

Lead Assembly 150 can comprise one or more elongate filaments, leads 151, each including one or more electrodes and/or other sensors, sensor 160. Each lead 151 can comprise a flexible filament, and can be configured to be under the patient's skin, at one or more internal body locations of the patient. In some embodiments, one, two, or more leads 151 can be positioned above the patient's skull, such that one or more sensors 160 of each lead 151 can produce a signal representative of electrical activity of the brain. In these embodiments, each sensor 160 can be positioned on the top of the skull, within the bone of the skull, or through the skull, each as described in detail herebelow. In some embodiments, one or more leads 151 can be positioned below the temporal muscle (e.g. in a location between the temporal muscle and the temporal bone). This sub-temporal muscle location can be used to reduce artifacts in signals produced by sensors 160, such as when the side of sensor 160 (e.g. an electrode) facing the temporal muscle is shielded, and the side of sensor 160 facing the brain is not shielded.

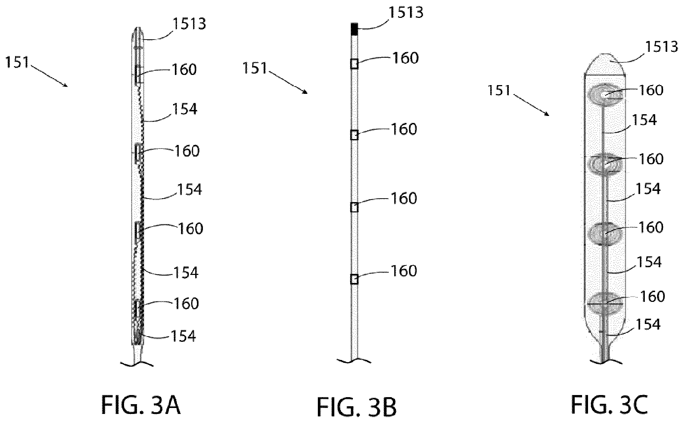

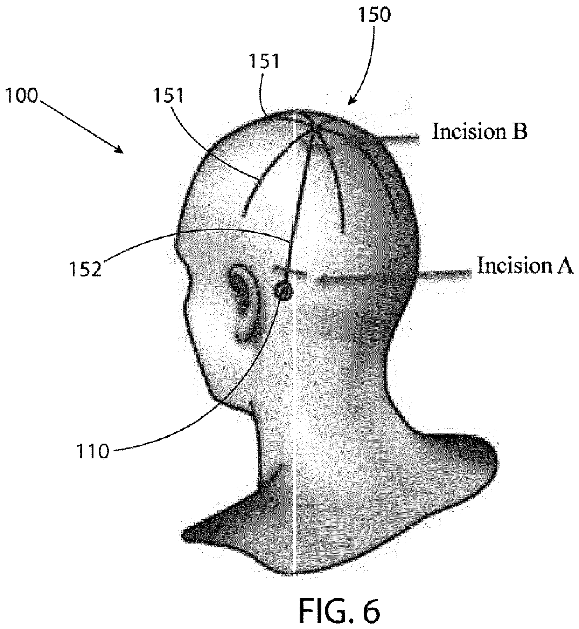

Each lead 151 can comprise one or more sensors 160. In some embodiments, lead assembly 150 is constructed and arranged as described herebelow in reference to Figs. 3A-C, 4A-C, and/or 5A-B. In some embodiments, lead assembly 150 is implanted as described herebelow in reference to Fig. 6.

In some embodiments, lead assembly 150 comprises multiple leads 151 that can be implanted in a star-shaped arrangement, such as is described herebelow. In some embodiments, lead assembly 150 comprises at least two leads, at least three leads, at least four leads, at least five leads, at least six leads, at least eight leads, and/or at least ten leads. Leads 151 can be implanted (e.g. in a star shape or other geometry) to monitor activity (e.g. electrical activity) of one or more portions of the patient's brain, placed via one or more small incisions in the patient's skin, without requiring a craniotomy. In some embodiments, lead assembly 150 comprises multiple leads 151 whose geometry when implanted on the patient's skull defines a convex hull that covers at least 10%, at least 50%, or at least 75%, or up to 100% of the convexity of the cerebral hemisphere (e.g. the cerebral convexity directly facing the cranial vault).

Each lead 151 can be tunneled through tissue (e.g. subcutaneous tissue) individually or in combination with one or more other leads 151. Lead assembly 150 can be operably connected (e.g. at least electrically connected) to ITX 110 via one or more flexible wires or traces (e.g. conductors 154 described herein) positioned within conduit 152 shown. Lead assembly 150 can be pre-attached to ITX 110, and/or it can be operably connected to ITX 110 by the implanting clinician (e.g. during the implantation procedure). Leads 151 can be distributed (e.g. radially distributed) over all or a portion of the patient's skull, using subcutaneous tunneling techniques via a small number of skin incisions as described herebelow. In some embodiments, leads 151 are distributed over all or a portion of the patient's skull via one or two small incisions, such as incisions of no more than 2cm.

Each lead 151 can comprise one or more sensors 160, such as between three and twelve sensors 160 (e.g. electrodes), or between four and five sensors 160 (e.g. electrodes).

Each lead 151 can comprise a relatively linear shape with a width less than or equal to 5mm, such as less than or equal to 3.5mm. In some embodiments, lead 151 comprises concentric electrodes (e.g. tripolar concentric electrodes), and lead 151 has a width less than or equal to 12mm, or 10mm. In some embodiments, lead 151 comprises tubular sensors (e.g. tubular electrodes), and lead 151 has a width less than or equal to 2.5mm, 2.0mm, or 1.5mm. Each lead 151 can comprise a length of at least 5cm, or a length of no more than 15cm, or a length of approximately 10cm.

Leads 151 can each comprise a substrate on which sensors 160 are mounted. The substrate of lead 151 can have a relatively flat cross section, a relative round cross section, and/or it can include segments with relatively flat cross section and segments with relatively round cross section (e.g. tubular segments, or semi-tubular). Lead 151 can comprise one or more biocompatible materials, such as silicone, polydimethylsiloxane (PDMS) polymer, and/or polycaprolactone. In some embodiments, at least a portion of lead assembly 150 comprises a biodegradable material (e.g. a biodegradable polymer), as described herebelow. Leads 151 can be reinforced with a coating (e.g. a parylene coating), reinforced with a mesh (e.g. a metallic or plastic mesh), or reinforced with one or more filaments (e.g. suture).

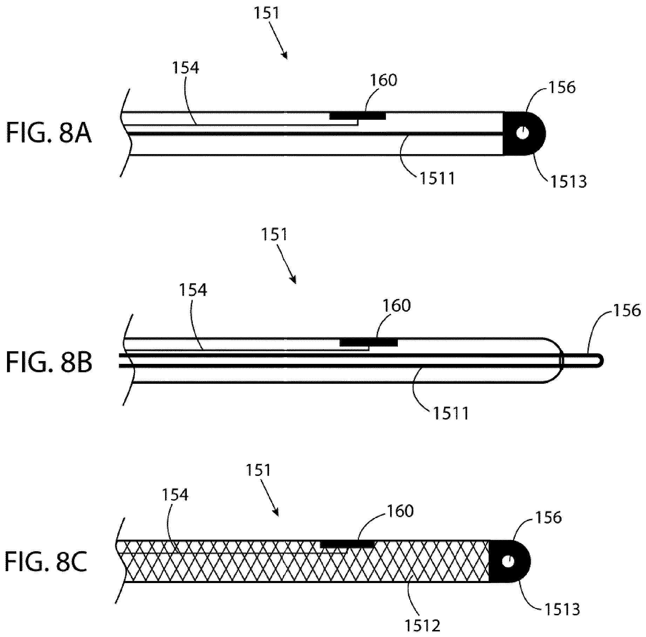

In some embodiments, leads 151 can be reinforced, such as is described herebelow in reference to Figs. 8A-B. The reinforcing of lead 151 can support forces (e.g. stretching, compressing, and/or twisting) encountered during implantation (e.g. tunneling) or explantation, and/or those forces encountered during washing, scratching, and other daily life activities (forces incurred while implanted that are caused by relative movement of the tissue planes between which leads 151 are implanted). Each lead 151 can comprise a distal portion (e.g. a tip) that is configured to be engaged by one or more tunneling tools without damage (e.g. including a material with sufficient durability and/or a reinforced material), such as tip 1513 described herebelow in reference to Figs. 8A-B

In some embodiments, one or more leads 151 comprises an attachment element configured to engage an introducer and/or a tunneling tool, such as introducer tool 810 described herein. For example, a lead 151 can comprise attachment element 156 described herebelow in reference to Figs. 8A-B.

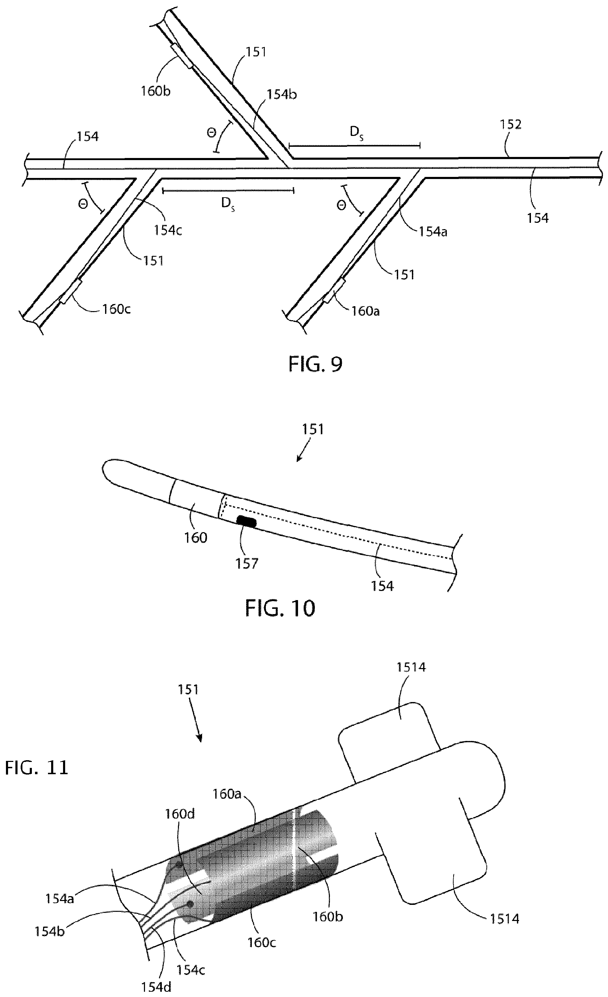

In some embodiments, one or more leads 151 comprise one or more markers, such as when a marker is positioned proximate or otherwise relative to one, two or all sensors 160 of that lead 151. For example, lead 151 can comprise marker 157 described herebelow in reference to Fig. 10, such as when tool 800 comprises an imaging device configured to provide an image of marker 157, to assist in locating the referentially positioned sensor 160.

In some embodiments, one or more portions of leads 151 and/or another portion of lead assembly 150 comprises a coating, such as a parylene coating (e.g. a parylene C coating). In some embodiments, one or more portions of leads 151 and/or another portion of lead assembly 150 comprises a coating configured to reduce tissue adhesion, such as to ease explantation of lead assembly 150. In some embodiments, lead assembly 150 comprises a coating including a drug or other agent to be eluded into the patient over time.

In some embodiments, lead assembly 150 comprises multiple leads 151 positioned in a staggered arrangement, such as is described herebelow in reference to Fig. 9.

As described above, sensor 160 can comprise one or more electrodes and/or one or more other sensors. In some embodiments, sensor 160 comprises one, two, or more sensing elements selected from the group consisting of: electrical activity sensor; electrode; magnetic sensor; light sensor; pressure sensor; force sensor; strain gauge; motion sensor; vibration sensor; accelerometer; gravimetric sensor; pH sensor; temperature sensor; humidity sensor; physiologic sensor; blood pressure sensor; pulse sensor; blood sensor; blood gas sensor; glucose sensor; ion sensor; small molecule sensor; steroid sensor; protein sensor; respiration sensor; and combinations thereof. Each sensor can be connected to one or more wires or other filaments, such as to transfer information and/or energy to and/or from ITX 110.

In some embodiments, sensor 160, and/or another sensor of the system as described herein, comprises one or more vibration sensors arranged and configured (e.g. positioned) to produce signals to detect speech and/or other utterances ("speech" herein) of the patient. These signals can be used to simply detect that the patient is speaking and/or they can be used to determine specific words spoken by the patient. In some embodiments, sensor 160 comprises a sensor (e.g. a vibration sensor) used to detect ictal crying of the patient. Sensor 160 can comprise one or more vibration sensors comprising one or more sensors that engage (e.g. screw into) the patient's skull. In these embodiments, sensor 160 of system 10 can comprise one or more vibration sensors, and one or more other sensors (e.g. one or more electrodes configured to record brain activity of the patient).

In some embodiments, sensor 160 comprises one or more electrodes, such as platinum-iridium electrodes.

In some embodiments, sensor 160 comprises a tubular sensor (e.g. a tubular electrode), such as is described herebelow in reference to Fig. 10.

In some embodiments, sensor 160 comprises multiple electrodes positioned circumferentially around one or more segments of a lead 151, such as the facet electrodes described herebelow in reference to Fig. 11. Each facet electrode can span a partial circumferential segment, such as a segment of no more than 180°, or no more than 90°, or no more than 60°.

In some embodiments, sensor 160 comprises a transducer, such as a stimulation delivery element (e.g. an element configured to deliver stimulation energy). In these embodiments, sensor 160 can also be configured as a sensor, such as when sensor 160 comprises one or more electrodes configured to record electrical activity in tissue, and deliver electrical energy to tissue. In some embodiments, sensor 160 is configured to provide stimulation by delivering to tissue a form of energy selected from the group consisting of: electrical energy; magnetic energy; electromagnetic energy; light energy; chemical energy; thermal energy (e.g. heating and/or cooling); sound energy such as subsonic and/or ultrasonic sound energy; mechanical energy; and combinations of these. In some embodiments, sensor 160 is configured to deliver a pharmaceutical drug or other agent to the patient (e.g. an agent to treat epilepsy or other brain condition). Energy (e.g. stimulation energy) can be delivered to various tissue locations of the patient, such as brain tissue (e.g. cortex of the brain, deep brain nuclei) and other nerve tissue (e.g. vagal nerve tissue).

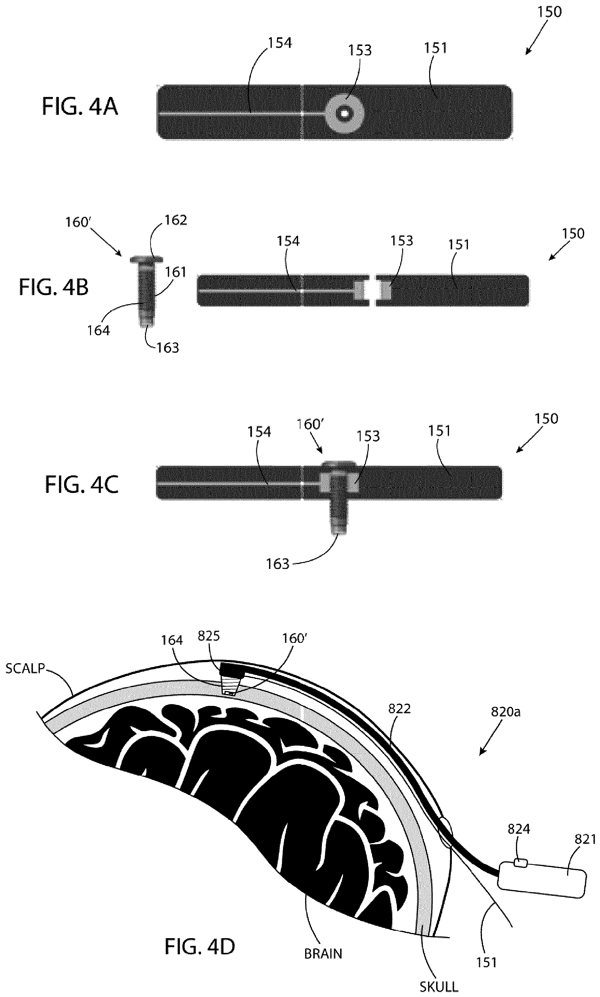

In some embodiments, sensor 160 comprises one or more sensors inserted into the skull (e.g. an intra-bone skull electrode), such as through the skull, such as sensor 160' described herebelow in reference to Figs. 4A-D.

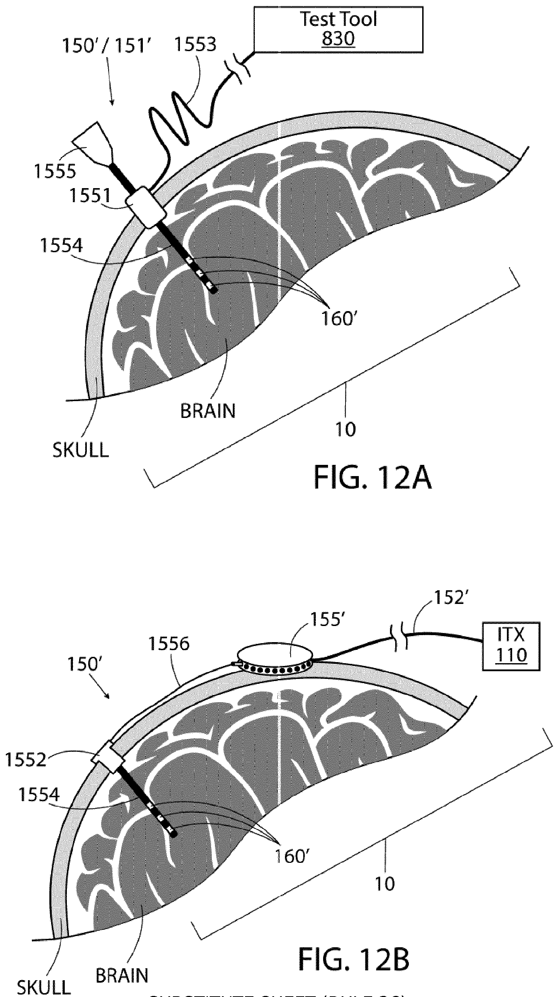

In some embodiments, sensor 160 comprises an intracranial sensor, such as an subdural electrode (strip, and/or grid), or a penetrating depth electrode, such as is described herebelow in reference to Figs. 12A-B.

In some embodiments, sensor 160 comprises one or more optical sensors, such as one or more functional near infrared spectroscopy (fNIRS) sensors, The one or more optical sensors can be positioned into and/or through the patient's skull (e.g. within the cranium). The one or more optical sensors can be configured to produce a signal used to measure cerebral hemodynamics within the patient's brain.

In some embodiments, sensor 160 comprises one or more foramen ovale electrodes, which can be placed at one or more body locations, such as through the skull (e.g. through a natural opening in the skull). The one or more foramen ovale electrodes can be positioned to measure physiologic parameters related to mesial temporal lobe activity (e.g. related to epilepsy or other brain condition). In some embodiments, sensor 160 comprises one or more sphenoidal electrodes configured to measure one or more mesio-temporal physiologic parameters.

In some embodiments, lead assembly 150 comprises between 1 and 64 sensors 160 (e.g. between 1 and 64 electrodes). In some embodiments, each lead 151 comprises between 1 and 16 sensors 160 (e.g. between 1 and 16 electrodes) . In some embodiments, lead assembly 150 comprises at least 6 sensors 160 (e.g. at least 6 electrodes).

In some embodiments, one or more sensors 160 (e.g. one or more electrodes), or another portion of lead assembly 150 comprises shielding material (e.g. at least a portion of an electrode is shielded), such as electromagnetic shielding material configured to reduce artifacts in the signals produced by sensor 160. For example, sensor 160 can comprise shield 165 shown, which can be positioned on one or more surfaces (e.g. a flat side) of sensor 160 that is to be positioned away from the brain or other area of recording and/or stimulation (e.g. positioned toward a source of interference such as a muscle, such as the temporal muscle as described hereabove).

As described hereabove, ITX 110 is configured to wirelessly transmit information through the skin to ETX 210 of external device 200. For example, ITX 110 can transmit information as recorded by lead assembly 150, and/or information that is derived from the recorded information (e.g. as processed by IPU 120 described herebelow). In some embodiments, ITX 110 is configured to wirelessly transmit information to other components of system 10, as described herein. In some embodiments, ITX 110 is configured to receive commands and/or other information (e.g. ITX 110 is configured as a transceiver for both transmitting and receiving information), such as information transmitted by external device 200 or another component of system 10.

ITX 110 can receive the information to be transmitted directly from lead assembly 150 (e.g. electrical activity or other signals from sensors 160), and/or from another component of implantable device 100, such as IPU 120 described herebelow.

ITX 110 can be implanted at one or more locations under the patient's skin, such as at a location behind the patient's ear and/or on the patient's chest and/or at the cranial vertex. ITX 110 can be implanted in the patient as described herebelow in reference to Fig. 6.

In some embodiments, implantable device 100 includes an **implantable processing unit, IPU 120,** which can be configured to process data, such as data including information received via signals produced by one or more sensors 160. Data processing performed by IPU 120 includes but is not limited to: amplifying; referencing; re-referencing; mathematically processing; digitizing; condensing; compressing; notch filtering; band-pass filtering; scaling; zero-centering; averaging; determining a maximum; determining a minimum; determining a mean; thresholding; transforming; spectrally analyzing; integrating; differentiating; performing signal conditioning; feature extraction; and combinations thereof. In some embodiments, IPU 120 is configured to perform feature extraction (e.g. temporal, spectral, wavelet and/or other feature extraction), and subsequently apply a classification regression and/or other machine learning algorithm (e.g. an artificial neural network) on the extracted features. In some embodiments, IPU 120 is configured to perform a time series analysis of recorded data, such as: auto-correlation; cross-correlation; stochastic process analysis; and/or chaotic time series analysis. In some embodiments, IPU 120 is configured to transform the time series analysis of the recorded data to a different domain, such as: spectral; wavelet; chirplet, and the like, and/or to apply another representation such as a generalized Fourier Transform. IPU 120 can apply classification regression, deep learning, reinforcement learning, and/or other machine learning algorithms to the processed data. In some embodiments, IPU 120 comprises memory storage circuitry configured to store information, such as information recorded by sensors 160, information received from a separate component of system 10 such as external device 200, and/or information processed by IPU 120.

In some embodiments, ITX 110 and IPU 120 are positioned in a single housing, such as is described herebelow in reference to Fig. 2.

In some embodiments, IPU 120 is configured to provide energy and/or an agent to one or more sensors 160, such as when one or more sensors 160 are configured as a stimulation element. For example, an electrode based sensor 160 can be configured to record electrical activity and/or deliver electrical energy, such as in a monopolar or multipolar arrangement as described herein. In some embodiments, IPU 120 comprises a reservoir (e.g. a refillable reservoir), which can be used to deliver a drug or other agent via one or more agent-delivery-based sensors 160.

In some embodiments, implantable device 100 includes an **implantable energy storage assembly, IESA 140,** which stores energy such as electrical energy. IESA 140 can comprise one or more batteries and/or capacitors, and it can be configured to be recharged, such as via energy received from an external device of system 10, such as via external device 200 as described herebelow. IESA 140 can provide energy to one or more components of implantable device 100. In some embodiments, IESA 140 provides energy to electronic componentry of IPU 120. In some embodiments, IESA 140 provides energy to one or more stimulation-delivering components of implantable device 100, such as one or more electrode-based sensors 160 and/or electrode-based functional elements 199 (e.g. an electrode positioned on the skull of the patient, in brain tissue of the patient, and/or at another patient location).

In some embodiments, ITX 110, IPU 120, and/or IESA 140 are positioned in a single housing, such as is described herebelow in reference to Fig. 2.

As described above, implantable device 100 can be figured to stimulate tissue of the patient, such as to stimulate brain tissue to achieve neuromodulation or otherwise treat or assess a medical condition of the patient. Implantable device 100 can include one or more stimulation delivery elements (e.g. sensors 160 and/or functional elements 199), such as one, two or more stimulation delivery elements selected from the group consisting of: electrode; energy delivery element; electrical energy delivery element; magnetic energy delivery element; light delivery element; sound delivery element; ultrasound delivery element; agent delivery element (e.g. a pharmaceutical drug delivery element); and combinations thereof. System 10 can be configured to deliver stimulation energy from these stimulation elements in order to perform "neuroprobing", as described herein, or to provide a therapeutic benefit to the patient (e.g. stimulation delivered based on the recording of the patient's brain activity and/or other physiologic parameters).

In some embodiments, one or more portions of implantable device 100 is biodegradable, such as one or more portions of leads 151, sensors 160, lead assembly 150, or a ITX 110 (e.g. a housing of ITX 110). In some embodiments, a shaft of lead 151 and/or a stimulation element of system 10 comprises one or more biodegradable portions. For example, one or more portions of implantable device 100 (or other implantable device of system 10 such as second device 900) comprises a biodegradable material such as a biodegradable metal (e.g. magnesium) and/or a biodegradable plastic (e.g. a biodegradable polymer, a conductive biodegradable polymer, polycaprolactone, Pedot-PSS, and/or a biodegradable polyester). In some embodiments, all or a portion of lead assembly 150 is biodegradable, such as to avoid explantation of all or a portion of lead assembly 150 after device use is completed or a device failure is detected. In some embodiments, biodegradation is initiated and/or assisted with the delivery of an agent or energy configured to biodegrade the particular component(s), such as when system 10 comprises an energy or agent delivery assembly, as described herein, that is configured to deliver the biodegradation agent and/or energy. In some embodiments, system 10 includes the agent to be delivered to assist in the biodegradation.

External device 200 can comprise one or more discrete components (e.g. one or more discretely housed components). At least one component of external device 200 is positioned proximate the patient's skin, e.g. about the patient's head or otherwise at a location proximate a portion of implantable device 100 (e.g. proximate to ITX 110).

As described hereabove, external device 200 includes ETX 210 which is configured to wirelessly receive information from ITX 110 of implantable device 100. In some embodiments, ETX 210 is configured to receive wireless radio frequency (RF) communications. In some embodiments, ETX 210 is configured to wirelessly receive information from other components of system 10, as described herein. In some embodiments, ETX 210 is further configured to transmit commands and/or other information (e.g. ETX 210 is configured as a transceiver for both transmitting and receiving information, such as via RF communications), such as information transmitted to implantable device 100 or another component of system 10.

In some embodiments, ETX 210 is further configured to transmit power (e.g. power and data) to implantable device 100, such as power transmitted via RF transmission and/or inductive coupling with implantable device 100, such as to store energy in IESA 140.

In some embodiments, external device 200 includes a processing unit, EPU 220, which can be configured to process data, such as data including information received from implantable device 100 and/or another component of system 10. Data processing performed by EPU 220 includes but is not limited to: amplifying; referencing; re-referencing; mathematically processing; digitizing; condensing; compressing; notch filtering; band-pass filtering; scaling; zero-centering; averaging; determining a maximum; determining a minimum; determining a mean; thresholding; transforming; spectrally analyzing; integrating; differentiating; performing signal conditioning; feature extraction; and combinations thereof. In some embodiments, EPU 220 comprises memory storage circuitry configured to store information, such as information received from implantable device 100 or another component of system 10, and/or information processed by EPU 220.

In some embodiments, external device 200 includes user interface 230, which can include one or more user interface components, as described hereabove. For example, external device 200 can include a button or other user input component such that the patient can provide feedback to system 10 via external device 200 (e.g. for the patient to indicate their detection of a neurological event such as a seizure). User interface 230 can include a vibrational transducer and/or other alert element such that an alert or other information can be provided to the patient or other user via external device 200.

In some embodiments, external device 200 includes an **energy storage assembly, ESA 240,** which stores energy such as electrical energy. ESA 240 can comprise one or more batteries and/or capacitors, and it can be configured to be recharged, such as via energy received from an external device of system 10, such as via a standard wall outlet charger. ESA 240 can provide energy to one or more components of external device 200. In some embodiments, energy from ESA 240 is wirelessly transmitted to implantable device 100, such as using inductive coupling or RF power transmission techniques.

In some embodiments, one or more portions of external device 200 is configured to be attached to a patient garment, such as a strap, belt, hat, headband, and/or glasses. In some embodiments, one or more portions of external device 200 can include a clip or other attachment element to releasably engage to a patient garment and/or the patient's skin. In some embodiments, one or more portions of external device 200 is positioned in a pocket of a garment worn by the patient. In some embodiments, one portion of external device 200 is positioned proximate the patient (e.g. ETX 210 is positioned proximate ITX 110, such as at a location proximate the patient's ear), while another portion of external device 200 (e.g. EPU 220, user interface 230 and/or ESA 240) is positioned proximate another location on the patient (e.g. the patient's waist) or at a location somewhat remote from the patient (e.g. a table or chair).

In some embodiments, system 10 comprises one or more controllers, **controller 300,** such as **controllers 300a and/or 300b** shown, each of which can be configured to send commands and/or receive information from external device 200 and/or implantable device 100. In some embodiments, controller 300b is configured for use by the patient, and controller 300a is configured for use by a clinician or other administrator of system 10. Patient commands enabled by a controller 300b can be a subset of the clinician commands enabled by a controller 300a, such as when the patient enabled commands of a controller 300b are a set of commands pre-approved for patient use by the patient's clinician.

Controller 300 can comprise **CTX 310** which can be configured to transmit and/or receive information, such as information transmitted and/or received to and/or from implantable device 100, external device, 200, and/or server 500 (e.g. via network 600).

Controller 300 can comprise **user interface 330,** which can include various user interface components, as described hereabove, to receive information from a user of system 10. Patient controller 300b can be configured to record information from the patient (e.g. patient-provided feedback information), as described herein, such as patient-provided information that is included in data analysis performed by one or more algorithms 50 of system 10.

In some embodiments, user interface 330 provides information related to signals recorded by sensors 160 and/or other sensors of system 10. For example, user interface 330 can provide, to a clinician and/or the patient, information related to (e.g. a representation or analysis of) brain signals of the patient. The information can be provided visibly (e.g. graphically and/or textually), audibly, and tactilely to the patient or other user. The information can be provided in real time or near-real time ("real time" herein), such as when epileptic or other information is provided to a patient in a neurofeedback therapy arrangement.

In some embodiments, system 10 includes a computer network, network 600, such as the internet and/or a privately maintained computer network. Network 600 can include wired and/or wireless connections, and can operably connect two or more components of system 10 (e.g. such that information can be transferred between two or more components of system 10).

In some embodiments, server 500 is a data logging module which can receive information from one or more components of system 10, such as external device 200, controller 300, and/or another component of system 10. Information can be transmitted to server 500, such as via network 600, continuously and/or intermittently (e.g. a predetermined intervals). Information can be transmitted and logged on a long-term basis, such as for a period of at least 1 week, at least 1 month, at least 6 months, or at least one year, and more.

Information transmitted to server 500 can include physiologic parameter information recorded by implantable device 100, second device 900, and/or another component of system 10, such as brain activity information and other physiologic information of the patient, as described herein. Logged information can include information recorded by implantable device 100 and/or second device 900, described herebelow. Logged information is stored on an information "cloud" or other database, cloud 550. An information processing module, global processing unit GPU 510 can analyze the information, such as via one or more algorithms 50. Analysis can be performed in an automated, semi-automated, and/or manual manner, such as with or without the involvement of a clinician or other user of system 10. Cloud 550 can include information from a single patient that can be analyzed via an algorithm 50 of GPU 510, such as to assess changes in that patient's condition, such as to determine improvement and/or worsening of the condition (e.g. to assess the effectiveness of a therapy). GPU 510 can include automated and/or semi-automated data-analysis software, data visualization tools, and/or graphics with summary statistics (e.g. statistics that are accessible by medical providers and patients).

In some embodiments, GPU 510 can perform a data analysis and/or creation function selected from the group consisting of: dataset selection; data "cleaning" and preprocessing (e.g. artifact removal, noise removal, and the like); data reduction; selection of an algorithm for processing; pattern extraction; data evaluation and/or interpretation; report generation; and combinations of these.

In some embodiments, system 10 includes multiple implants 100 that are implanted in multiple patients. In these embodiments, multiple external devices 200 and other associated components of system 10 can also be provided to each patient, and server 500 can collect sensor 160 and other associated information from multiple patients, such as to analyze, via one or more algorithms 50, data from a population of multiple patients stored on cloud 550, to provide system-provided feedback (e.g. neurofeedback) to one or more patients. Cloud 550 can include information from multiple patients that can be analyzed (e.g. by an algorithm 50 of GPU 510) to assess group similarities and dissimilarities, and/or to compare changes in patient conditions within a group (e.g. to discriminate the effects of therapy between multiple different therapeutic regimes used among the group). Analysis of single or multiple patient data stored in cloud 550 can be analyzed by an algorithm 50 in an automated or semi-automated manner.

System 10 can include one or more algorithms, algorithm 50, which can be used to perform an assessment or other function, as described herein. Algorithm 50 can be included, in whole or in part, embedded in a single component, or in multiple components, of system 10. In some embodiments, a first algorithm 50 is included in an implanted component of system 10 (e.g. included in implantable device 100), and a second algorithm 50 is included in an external component of system 10 (e.g. external device 200, controller 300, and/or server 500).

In some embodiments, system 10 includes one or more tools, such as **tools 800** shown.

Tool 800 can comprise **introducer tool 810** which can be configured to tunnel one or more leads 151 of lead assembly 150 under the patient's skin, such as a tunneling of a single lead 151, and/or multiple leads 151 (simultaneously) that are tunneled in subcutaneous tissue above the skull of the patient, as described herebelow. Introducer tool 810 can comprise forceps, or other grasping tool configured to engage a distal portion of one or more leads 151, such as to tunnel lead 151 through tissue (e.g. subcutaneous or other tissue). Tool 810 can comprise forceps relatively long, thin, curved, and/or malleable forceps, and can include an engagement portion for connecting to one or more attachment elements of lead 151 as described herein. Introducer tool 810 can comprise a narrow tube configured to surround and engage one or multiple leads 151, and to subsequently be tunneled through tissue to deliver the one or more leads 151 to a destination. Introducer tool 810 can comprise a tool which is configured to releasably engage an attachment element of one or more leads 151. In some embodiments, introducer tool 810 comprises a first introducer tool 810a configured to create a tunnel through tissue, and a second introducer tool 810b configured to engage a distal portion of one or more leads 151 to tunnel those one or more leads through the tissue tunnel previously created by first introducer tool 810a.

Tool 800 can comprise **sensor rotation tool 820,** a tool configured to engage and rotate a sensor 160, such as to insert a sensor 160 into the skull or other bone location. Sensor rotation tool 820 can comprise axial rotation tool 820b, an axial rotation tool (e.g. a straight screwdriver tool) configured to rotate a sensor 160 via an incision proximate and above the bone insertion location. Alternatively, sensor rotation tool 820 can comprise right-angle rotation tool 820a, a right-angle rotation tool configured to rotate a sensor 160 via an incision remote from the bone insertion location, such as right-angle rotation tool 820a described herebelow in reference to Fig. 4D.

Tool 800 can comprise **test tool 830,** a tool configured to temporarily record from one or more sensors of system 10, and/or deliver energy (e.g. electrical energy) to one or more stimulation elements of system 10. Test tool 830 can be used in the clinical setting (e.g. during implantation of implantable device 100) in a titration procedure that allows an operator (e.g. a clinician of the patient) to configure (e.g. set or adjust) one or more operator adjustable parameters (e.g. sensor position, recording setting, and/or stimulation level).

Tool 800 can comprise **one or more other tools,** such as a surgical drill (e.g. to drill pilot holes for screw-based sensors); a raspatory tool (e.g. to make an initial tunnel in tissue to ease subsequent tunneling); a trocar; an imaging device (e.g. a fluoroscope, X-ray imager, ultrasound imaging device, magnetic field imaging device, and/or infrared camera); and combinations of these.

In some embodiments, system 10 comprises an additional diagnostic and/or therapeutic device, second device 900. Second device 900 can be implanted in the patient, such as is shown in Fig. 1, or it can remain external to the patient. Second device 900 can stimulate patient tissue, such as to treat a patient condition based on patient physiologic information recorded by implantable device 100 (e.g. by one or more sensors 160 or other sensors of implantable device 100). In some embodiments, implantable device 100 adjusts stimulation in a closed-loop arrangement in which agent delivery is adjusted dynamically based on recordings made by sensors 160 and/or other sensors of system 10 as described herein.

Second device 900 can comprise a transceiver (e.g. transceiver 910 shown), a processing unit (e.g. processing unit 920 shown), a lead assembly (e.g. lead assembly 950 shown, comprising one or more leads 951), an energy storage assembly (e.g. energy storage assembly 940 shown), and a sensor (e.g. sensor 960 shown). Transceiver 910, processing unit 920, lead assembly 950, energy storage assembly 940, and sensor 960 can be of similar construction to ITX 110, implantable processing unit 120, implantable lead assembly 150, implantable energy storage assembly 140, and sensor 160, respectively, each as described hereabove. Second device 900 can comprise functional element 999, such as one or more sensors, transducers, or other functional elements as described herebelow.

Second device 900 can include one or more stimulation delivery elements (e.g. sensors 960 and/or functional elements 999), such as one, two or more stimulation delivery elements selected from the group consisting of: electrode; energy delivery element; electrical energy delivery element; magnetic energy delivery element; light delivery element; sound delivery element; ultrasound delivery element; agent delivery element; and combinations thereof. System 10 can be configured to deliver stimulation energy from these stimulation elements in order to perform neuroprobing, as described herein, or to provide a therapeutic benefit to the patient (e.g. stimulation delivered based on the recording of the patient's brain activity and/or other physiologic parameters).

In some embodiments, second device 900 comprises a drug or other agent delivery pump, such as an implanted or external agent delivery pump. In these embodiments, system 10 can automatically initiate and/or adjust ("adjust" herein) agent delivery, such as in a closed-loop arrangement in which agent delivery is adjusted dynamically based on recordings made by sensors 160 and/or other sensors of system 10 as described herein. Second device 900 can comprise a constant flow or adjustable (e.g. programmable rate pump). Second device 900 can comprise a pump with an attached delivery catheter that can be positioned at one or more locations within the patient, such as to deliver an agent to a location selected from the group consisting of: subcutaneous space; subdural space; a cerebral ventricle; brain parenchyma; intravenous location; intraarterial location; and combinations of these.

In some embodiments, implantable device 100, external device 200, controller 300, and/or second device 900 comprises one or more sensors, transducers, and/or other functional elements, such as functional elements 199, 299, 399, and/or 999, respectively. In some embodiments, system 10 includes one or more other functional elements 99, such as functional element 99a implanted in the patient, and/or functional element 99b positioned on the skin, positioned proximate the patient, and/or otherwise positioned outside of the patient. In some embodiments, lead assembly 150 comprises one, two or more functional elements 199 (e.g. one or more transducer and/or sensor-based functional elements).

Functional elements 99, 199, 299, 399, and/or 999 can comprise a sensor, such as one, two, or more sensors selected from the group consisting of: accelerometer; motion sensor; pressure sensor; gravimetric sensor; magnetic sensor; force sensor; strain gauge; temperature sensor; humidity sensor; light sensor; physiologic sensor; and combinations thereof. The at least one sensor can comprise one, two, or more physiologic sensors selected from the group consisting of: electrical activity sensor; electroencephalogram (EEG) sensor; local field potential (LFP)sensor; an electrocorticogram (ECoG) sensor; an electromyogram (EMG) sensor; action potential spike sensor; glucose sensor; pressure sensor; blood gas sensor; blood pressure sensor; pulse sensor; ion sensor; small molecule sensor; steroid sensor; protein sensor; pH sensor; galvanic skin response sensor; electrodermal sensor; temperature sensor; electrocardiogram (ECG or EKG) sensor; respiration sensor; a sphenoidal electrode; a lactate sensor; and combinations thereof. The at least one sensor can comprise at least one brain activity sensor. The at least one brain activity sensor can comprise one, two, or more sensors selected from the group consisting of: EEG Sensor; LFP Sensor; intracranial EEG sensor; neuronal activity sensor; action potential spike sensor; multiunit sensor; and combinations thereof. The at least one sensor can comprise a sensor configured to be positioned within the cranium, and/or on top of the patient's skull. The at least one sensor can comprise at least one body position sensor. The at least one body position sensor can comprise at least one head position sensor. The at least one sensor can comprise at least one body motion sensor (e.g. an accelerometer). The at least one body motion sensor can comprise at least one head motion sensor. The at least one body motion sensor can comprise at least one eye motion sensor. The at least one body motion sensor can comprise at least one hand motion sensor.

Functional elements 99, 199, 299, 399, and/or 999 can comprise a transducer, such as one, two, or more transducers as described hereabove. In some embodiments, functional elements 99, 199, 299, 399, and/or 999 comprises a stimulation element, such as an energy delivering stimulation element configured to deliver energy selected from the group of: electrical energy; magnetic energy; light energy; chemical energy; thermal energy (e.g. heating and/or cooling); mechanical energy; and combinations of these.

In some embodiments, functional elements 99, 299, 399, and/or 999 comprises a switch, such as a switch configured to record user input or a user command (e.g. patient input or a patient command).

In some embodiments, functional elements 99, 199, 299, 399, and/or 999 comprise an element configured to provide information (e.g. system-provided feedback information) to a user, such as the patient. For example, the functional element can be configured to provide an audible and/or tactile alert, such as to alert the patient of a current and/or future neurological event (e.g. a current or prognosed future seizure or other neurological event). In some embodiments, feedback is provided to the patient via an alert element of implantable device 100, such as an alert element of ITX 110.

In some embodiments, functional element 199 of implantable device 100 and/or functional element 999 of second device 900, and/or functional element 99a comprises one or more implantable sensors. For example, functional element 99a, 199 and/or 999 can comprise one or more foramen ovale electrodes that are positioned through the skull to extend into the cranium, such as to provide direct mesio-temporal recordings. Alternatively or additionally, sphenoidal electrodes remain extra-cranial but closer to the mesio-temporal structures such as to provide antero-temporal recordings. Alternatively or additionally, functional element 99a, 199 and/or 999 can comprise one or more subdural and/or depth penetrating electrodes, such as electrodes that penetrate inside brain parenchyma via millimetric cranial drill holes (for depth electrodes), or centimetric burr-holes (for epidural or subdural electrodes). Alternatively or additionally, functional element 99a, 199 and/or 999 can comprise one or more fNIRS sensors and/or other optical sensors, such as to measure cerebral hemodynamics.

In some embodiments, one or more functional elements 99, 199, 299, and/or 999 comprise a stimulation delivering element that provides stimulation by delivering to tissue a form of energy selected from the group consisting of: electrical energy; magnetic energy; electromagnetic energy; light energy; chemical energy; thermal energy (e.g. heating and/or cooling); sound energy such as ultrasound energy; mechanical energy; and combinations of these. Stimulation can be delivered to perform a therapy, and/or to perform a diagnostic (e.g. a neuroprobing diagnostic as described herein).

In some embodiments, functional elements 99, 199, 299, and/or 999 comprise a drug delivery element (e.g. when second device 900 comprises an implantable and/or external drug delivery pump, such as a pump including a refillable drug reservoir). In these embodiments, system 10 can deliver one or more drugs to treat one or more medical conditions as described herein. These one or more functional elements can be arranged and positioned to deliver a drug to one or more locations on and/or within the patient, such as an anatomical location selected from the group consisting of: the skin; the mouth or other gastrointestinal location; subcutaneous tissue; a vein; an artery; a muscle; the heart; the brain; a ventricle of the brain; below the dura above the brain; the spine; the epidural space; the intrathecal space; and combinations of these. In some embodiments, brain activity or other physiologic parameters recorded by implantable device 100 can be used to modify drug or other agent delivery (e.g. initiate, increase, and/or stop or at least decrease a rate of drug delivery). In some embodiments, an anti-epileptic drug is being delivered by system 10, and drug delivery is initiated and/or increased when a seizure is predicted (e.g. predicted to occur in the near future) and/or detected (e.g. detected as being currently present) by system 10. In some embodiments, a pain alleviating drug is delivered by system 10, and the drug delivery is initiated and/or increased when pain is predicted and/or detected by system 10. In some embodiments, a psychiatric medication is delivered by system 10, such as to treat a mental illness. In some embodiments, a neuropsychopharmacology drug is delivered by system 10, such as to treat one or more of: anxiety disorder; affective disorder; psychotic disorder; addiction; degenerative disorder; eating behavior; and/or sleep behavior. In some embodiments, an antidepressant is delivered by system 10. In some embodiments, an anesthetic (e.g. a local anesthetic) is delivered by system 10, such as to alleviate pain or other discomfort encountered during stimulation by system 10. In some embodiments, drug delivery by system 10 is stopped or at least decreased when an undesired physiologic condition is detected by system 10 (e.g. an arrhythmia, heart attack, loss of consciousness, or other undesired event is detected by system 10).

In some embodiments, as mentioned above, system 10 can include one or more functional elements, such as functional elements 99a and/or 99b, which can each comprise sensors (or sensor assemblies) configured to produce a signal. Functional element 99a can comprise an implanted sensor, and functional element 99b can comprise an external sensor, each as shown. Functional elements 99a and/or 99b (singly or collectively "functional element 99) can be configured to produce a signal that is transmitted to another component of system 10 via a wired or wireless connection. In these embodiments, system 10 can be configured to perform a diagnostic, therapeutic and/or other medical procedure utilizing information recorded by both sensors 160 and sensor-based functional elements 99. For example, functional elements 99 can comprise sensors such as: temperature sensors; pressure sensors; photoplethysmogram sensors for heart rate monitoring; accelerometers for assessing patient motor behavior such as gait, fatigue and/or falls monitoring; and/or a microphone for verbal annotations or commands (speech recognition) and recording of speaking and other vocalizations. The collective information can be analyzed by one or more components of system 10, such as external device 200, controller 300, and/or network 600. The collective information can be stored on cloud 550, such as when similar information is stored on cloud 550 for other patients, as described herein.

In some embodiments, system 10 includes multiple sensors (e.g. sensors 160, sensors 960, and/or functional elements 99a, 99b, 199, 299, and/or 399, are provided in a kit arrangement, with all or a subset of the sensors made available in system 10 to be implanted in and/or placed proximate the patient (e.g. as selected by a clinician of the patient). For example, a diagnosis (e.g. a preliminary diagnosis) of a patient can be made, and a particular set of one or more sensors provided in system 10 are selected for use based on that diagnosis. The set of sensors can be chosen to record brain activity or other physiologic information of the patient, as described herein.

As described hereabove, system 10 can be configured to record electrical activity of the brain, and/or other physiologic parameter information of the patient, such as to process the information to perform a medical procedure (e.g. a diagnostic, prognostic, and/or therapeutic procedure) on a patient, such as a patient with a brain disorder. For example, implantable device 100, second implantable device 900, and/or another component of system 10 can include one or more sensors (e.g. sensors 160) that record a physiologic parameter selected from group consisting of: neuronal activity; brain activity; activity in the brain cortex; activity in the deep brain; muscle activity; action potential activity; glucose levels; blood pressure; blood gas levels; pH of a body fluid; skin conductance; electrodermal activity; temperature (e.g. tissue or body fluid temperature); heart activity; respiration activity; and combinations thereof.

System 10 can be configured to perform a neurologic, psychiatric, and/or sleep medicine procedure. System 10 can be configured to perform neurorehabilitation, manage and/or treat pain, and/or function as an assistive technology to the patient (e.g. when configured as a brain-machine interface). System 10 can perform a medical procedure on a patient with abnormal electroencephalography (EEG) or other abnormal brain activity, such as a patient with epilepsy in which long-term monitoring of brain electrical activity is performed by system 10.

In some embodiments, system 10 is configured to diagnose, prognose, and/or treat, one, two, or more medical conditions selected from the group consisting of: brain condition; neurological condition; epilepsy; coma; psychiatric disorder; mood disorder; obsessive compulsive disorder; an attention deficit disorder; Tourette's syndrome; a neurogenerative disease; a movement disorder; essential tremor; Parkinson's disease; a tic disorder; sleep disorder; pain; neuropathic pain; stroke; paralysis; tinnitus; dyslexia; a speech production disorder such as aphasia; a memory disorder such as amnesia; chronic fatigue syndrome; chronic headaches; multiple sclerosis and other chronic demyelinating disorders and combinations of these.

In some embodiments, system 10 is configured to warn a patient of a neurological event, such as a seizure. System 10 can provide a warning (e.g. via a functional element as described herein) of a current event and/or a potential future event (e.g. a current seizure or a prognosed future seizure). For example, algorithm 50 of system 10 can analyze recorded brain information and/or other physiologic information to predict the occurrence (and/or determine a risk of occurrence) of a seizure or other neurological event (e.g. a current event or a future event). The assessed risk can be provided to the patient (e.g. via user interface 330 or otherwise). In some embodiments, if the assessed risk is above a pre-determined threshold (e.g. a clinician-determined threshold and/or a machine-learned or other machine-determined threshold), the patient is alerted.

In some embodiments, system 10 is configured to perform a medical procedure related to: stroke rehabilitation; paralysis including the Completely-Locked-In-Syndrome, CLIS (e.g. resulting from amyotrophic lateral sclerosis, ALS); chronic pain analgesia, particularly in neuropathic pain; coma and other brain disorders thought to result from dysfunction in brain circuits controlling emotion, memory, thought, or movement, such as psychiatric disorders including mood and anxiety disorders; tic disorders and obsessional-compulsive disorders including Tourette's syndrome; neurodegenerative diseases; movement disorders like essential tremor or Parkinson's disease; attention deficit disorders; sleep disorders; tinnitus; and/or dyslexia.

System 10 can include multiple sensors 160, such as up to 32, or up to 64 electrodes or other sensors. All or a portion of the sensors 160 can be placed under the skin and on top of and/or into the skull, such as to perform transcranial monitoring of EEG signals and other electrical and/or other physiologic activity from all or a portion of the patient's brain. The recordings can be performed (e.g. implantable device 100 can remain implanted) for at least one month, or at least one year. In addition to electrode-based sensors, one or more sensors 160 can comprise one or more fNIRS sensors, such as one or more intracranially placed optodes, as described herein. System 10 can monitor brain electrical activity as well as other physiologic parameters of the patient, in a multi-modal monitoring arrangement, such as to diagnose, prognose, and/or treat one or more medical conditions of the patient. This multi-modal monitoring can be used to provide a differential diagnosis, such as a differential diagnosis of epilepsy that monitors signals related to other disorders (e.g. to differentiate a cardiac event from an epileptic event).

System 10 can be configured to **assess global cerebral function of a patient through continuous physiological timeseries** (EEG, with concurrent heart rate measurement, electrodermal activity, and other physiological measures), such as an assessment performed over timescales of months to years. Today, monitoring and intervention in brain activity is generally accomplished by devices placed on the scalp or inside the skull. The former is problematic for long term monitoring because long term, continuous wear of an EEG cap is impractical, and the technology is cumbersome. The latter, which gives more stability and higher resolution for recording and/or stimulation functions, requires an extensive surgery (craniotomy) and often penetration of the brain. For these reasons, chronic intracranial device applications with brain recording capabilities are very rare, and non-practically existent for global brain coverage. System 10 addresses an unmet medical need for continuous and long-term access to electrophysiological activity of a patient's brain, with reliable physiologic parameter measurements (e.g. brain electrical activity measurement) and potentially stimulation (e.g. transcranial stimulation provided by sensors 160 and/or other brain tissue stimulation). System 10 can be used both within and outside of a hospital or other clinical setting. System 10 can avoid the need for a craniotomy, with significantly less invasive surgical placement than is offered by intracranial devices. System 10 offers to the patient minimal interference in daily life.

System 10 can be configured to provide continuous or relatively continuous ("continuous" herein) feedback to a patient. For example, user interface 330 and/or one or more transducers of system 10, as described herein, can be configured as a feedback assembly that alerts patients of a present or future neurological event, such as a seizure or other neurological event (i.e. the system provides "neurofeedback" to the patient). System 10 can be further configured to stimulate tissue of the patient, such as electrical stimulation delivered by implantable device 100 (e.g. transcranial stimulation delivered by sensors 160 or direct cerebral stimulation), second device 900, and/or another component of system 10.

System 10 can be configured as a **single or multi-patient disease management platform,** such as to diagnose, prognose, and/or treat a brain condition. Information recorded by implantable device 100 can be collected periodically and/or as a live stream, and processed. Information recorded by implantable device can be collected aperiodically, such as when information is recorded just prior to, during, and/or just after a neurological event such as a seizure. Processing of the information can be performed in part or in whole by: implantable device 100, external device 200, controller 300, server 500, and/or another component of system 10. System 10 can aggregate the information recorded by implantable device 100 and/or other recorded information as described herein, and compile long-term data-logs of disease activity and disease progression per patient and/or patient populations. System 10 can analyze the compiled data-logs in an automated and/or semi-automated arrangement (e.g. with inputs from health care professionals). System 10 can provide aggregated health reports (e.g. to clinicians of the patient and/or patients) to assist in data-driven, timely decisions, for a patient's therapy. This information can increase therapeutic efficiency, and enhance clinician interventions. System 10 can be configured as a **persuasive technology,** providing timely and data-driven information to the patient, such as to cause adoption of a health-promoting lifestyle and behavior.

System 10 can include multiple different sensors (e.g. sensor 160 of implantable device 100, sensor 960 of second device 900, and/or another sensor of system 10) that provide information to server 500, e.g. to cloud 550). The information can include at least EEG, ECG, and/or EMG information. Algorithm 50 can apply classifiers, filters, and/or other information processing. For example, arousal states can be assessed (e.g. automatically assessed by system 10) to create arousal-level time-series extracted from raw data. For example, system 10 can analyze (e.g. using mathematical processing, classification techniques, and the like) data that is transformed into a time-series that indicates a time course of one or more biomarkers (e.g. arousal level) which can then be monitored (e.g. over circadian or other cycles), and compared to neurological and other medical events (e.g. seizures). One layer of analysis provided by system 10 (e.g. via algorithm 50 and/or server 500) can be a statistical report of EEG features (e.g. continuously gathered EEG features such as power in a frequency band, epileptiform, and/or other pathological activity). Another layer of analysis can include taking all recorded variables as input features to define brain states at the multi-dimensional level. Recorded EEG can be enhanced with recordings from other physiological monitoring (e.g. as recorded by one or more sensors of system 10 as described herein). Brain states can be reflected in nearly all physiological variables, which allows not only the identification of temporal patterns in brain activity but also the correlation of these patterns with other physiological variables and rhythms (such as sleep, heart rate variability, body temperature, and the like) at multiple timescales, including the circadian cycle and the multi-day cycle. Subsequently, at both an individual patient level and for multiple patient populations, the combinatory influences (before any disease events) can be characterized. The characterization can be used to predict or (early) detect onsets of events (such as seizures). Using these techniques (e.g. long term recording of brain activity), system 10 can perform event forecasting, personalized chronotherapy (e.g. therapy scheduled in time), and dynamically adjust treatment dosing to specific states of the brain.

Storing of single or multiple patient information on cloud 550 can support a **data-driven decision-making process,** by making current and/or past information available when and where needed. Using system 10, clinicians can encourage and reward health-promoting patient behaviors and lifestyle improvements.

System 10 provides various novel medical interventions, such as improved diagnostics of brain conditions (e.g. brain diseases or disorders). System 10 can provide **continuous and long-term brain activity monitoring with data-logging,** as opposed to event-driven (e.g. after a seizure) or scheduled-based (e.g. once per year) short-term disease monitoring. System 10 can objectivize disease activity (e.g. seizure counts) over time and surpass unreliable patient self-reporting. System 10 can reveal infrequent as well as subclinical symptoms and allows the measurement of the rhythm of symptoms at multiple time scales (hours, days, weeks, months). The continuous data collection allows for early detection of anomalies and trend analysis on the health status of the patient, offering learning for improved treatment as well as the ability to assess the impact of ongoing interventions (e.g. are seizures less frequent when a new drug is given; are drugs becoming less effective). System 10 enables rigorous long-term monitoring enabling many novel therapeutic approaches that are complementary and increasing in sophistication. For example, informed clinical decisions are achieved as providers will base their clinical decisions on objective and accurate data on a given disorder (e.g. seizure count in epilepsy, sleep quality in sleep disorders) as opposed to patient reports known to be globally inaccurate, for example because brain disorders often come with cognitive dysfunction. Patient-based treatment decisions can be performed. Once proven accurate, information gathered by continuous monitoring via system 10 can be provided directly to the patient in a user-friendly format for their own decision-making. This approach to self-management in chronic disease already exists in diabetes for example, where patient learn to monitor glucose and adjust their insulin injections. System 10 can provide machine-learning treatment decisions. With the wealth of digital information gathered by system 10, machine-learning techniques that have proven successful for big-data mining in non-medical fields can be incorporated in algorithm 50 to enable therapeutic decision at the next level of integrative understanding. Conceptually, brain activity is reflected by a complex combination of multimodal variables (such as direct EEG measurements, and bodily effects such as heart rate, etc.) jointly defining a state-space. Within this space, some states relate to pathology, others to health. These states will define novel, complex biomarkers that can be tracked by system 10 to guide therapeutic interventions and dynamically improve health benefits.

System 10 can be configured to perform **"neuroprobing",** in which stimulation energy is delivered by one or more components of system 10, as described herein. For example, at least one electrode-based sensor 160 and/or one or more other stimulation delivery elements of system 10 (as described herein) can deliver stimulation energy to tissue (e.g. brain tissue) in a neuroprobing arrangement. The stimulation delivering element can be positioned subcutaneously on the skull of the patient (e.g. an electrode-based sensor 160 positioned on the skull) and/or it can be positioned on and/or in brain tissue (e.g. a stimulation element of implantable device 100 and/or second device 900 comprising stimulation delivering functional elements 199 and/or 999 respectively). Repetitive sub-threshold (i.e. not eliciting after-discharges) single-pulse electrical stimulation can be delivered (e.g. directly and/or indirectly to the cortex), with an aim to probe cortical excitability and record the magnitude of the evoked response. In some embodiments, transcranial stimulation is provided by one or more sensors 160 positioned on the top of the skull of the patient. Evoked response information (e.g. evoked response magnitude information) gathered by system 10 can comprise adjunct information of brain states that is complementary to passive multimodal recordings recorded by system 10. This stimulation provided by implantable device 100 or otherwise can function as a diagnostic tool, to assess cortical excitability. Stimulation can be provided by system 10 directly or indirectly to brain tissue (e.g. to the cortex of the brain), such as electrical energy delivered by one or more electrode-based sensors 160 of lead assembly 150 (e.g. positioned on skull beneath the skin), one or more scalp placed sensors (e.g. functional element 99b and/or other sensor placed on the scalp), and/or an intracerebral electrode (e.g. functional element 199, 999, and/or other sensor configured and positioned as an intracerebral electrode). Stimulation can be provided by multiple electrode-based sensors 160, such as when a first electrode is configured as a focal stimulation electrode, and one or more other electrode-based sensors 160 is configured as return electrodes. In these diagnostic configurations, system 10 can be used to determine state of disease in epilepsy, and/or it could be used to determine susceptibility to neurofeedback and/or neuromodulation therapies.

System 10 can be configured in a **"neurofeedback" mode.** For example, system 10 can provide self-administered, always available, neurofeedback (e.g. delivered as needed as opposed to scheduled-based neurofeedback currently available in the hospital/clinic setting). System 10 can provide neurofeedback that uses self-monitored readouts of the patient's brain activity to initiate behavioral, pharmacological, electrophysiological, and/or other interventions. Traditionally, neurofeedback involves learned self-regulation (by poorly understood mechanisms) of brain activity. System 10 can record brain signals continuously, providing information of interest to the patient, such as to enable the patient to take action without separate assistance. Patients may use internally perceived cues, but not directly measurable, to shape their own brain activity and alter their condition. For example, system 10, via patient controller 300b or external device 200, can provide feedback reporting the state of brain signals to the patient in real time. System 10 provides real-time, continuous, multiparametric (tele) monitoring of patient's brain activity, in combination with the physiological monitoring, in order to provide neurofeedback at the point and at the time of need, or based upon a patient request. Provided neurofeedback can be provided via visual displays, sound transducers, tactile (vibration) transducers, and/or combinations of these, as described herein. Always-available neurofeedback provides comfort to patients with chronic brain disorders since it is much less disruptive (or not disruptive at all) to their daily life, and does not require any laborious preparation (such as difficult and long application of scalp EEG electrodes). It has a positive impact on the healthcare system by reducing: costs per patient; number or duration of visits; costs for installations and maintenance of neurofeedback systems; and medical workflows.

System 10 can be configured in a **"neuromodulation" mode.** For example, system 10 can provide neuromodulation of brain circuits. System 10 can provide always-available stimulation of brain tissue (e.g. transcranial electrical stimulation delivered by sensors 160 and/or intracranial stimulation delivered by a stimulation element of system 10), which can be provided as needed, in a "responsive neuromodulation mode". The stimulation can be provided in a closed-loop arrangement. For example, stimulation can be delivered transcranially (e.g. stimulation delivered from stimulation elements positioned under the skin on the top of the skull), intracranially (e.g. stimulation delivered from stimulation elements positioned on and/or in brain tissue), or both transcranially and intracranially. System 10, including neuromodulation capabilities and brain signal recording (with real time analysis), allows real time adaptation of neuromodulation parameters to changing brain states. An always-available neuromodulation provides comfort to patients with chronic brain disorders since it is much less disruptive (or not disruptive at all) for their daily life and does not require any laborious preparation (such as application of scalp electrodes and system setup). It also allows as-needed neuromodulation interventions, as opposed to schedule-based neuromodulation sessions. System 10 can adjust stimulation or otherwise perform in a closed-loop arrangement, such as by reacting to detected abnormal brain activity and/or by adjusting to other information that is detected by system 10. In some embodiments, system 10 adjusts stimulation-driven neuromodulation based on monitored brain response to the neuromodulation (e.g. in a "closed-loop informed" arrangement).

System 10 can be configured to function as a **brain-machine interface (BMI),** such as when signals recorded by implantable device 100 (e.g. signals related to patient thoughts) are used to control a computer or other component of system 10. In some embodiments, system 10 is configured to operate as a BMI continuously, and for extended periods of time (e.g. at least one month, at least six months, and/or at least one year). For example, the patient may have a paralysis, such as completely-locked-in-syndrome (CLIS) resulting from amyotrophic lateral sclerosis (ALS), or any other etiology. System 10 can comprise a BMI that controls computers equipped with specialized applications, such as writing applications, home environment control applications, and the like. Configured as a BMI, system 10 provides access to brain signals that are always available.

System 10 can be configured to perform **"dynamic informed therapy" DIT,** wherein system 10 adjusts (e.g. continuously adjusts) a provided therapy (e.g. stimulation therapy, pharmaceutical drug therapy, and the like) to dynamic changes in the patient's neural state (e.g. as determined by an analysis performed by algorithm 50 of brain activity recorded by sensors 160 and/or other physiologic parameters recorded by system 10). For example, system 10 can gather information used to perform dynamic, informed, pharmaceutical drug and/or other titration, in which drug choice and/or dosage are guided by biomarkers of disease activity. In some embodiments, system 10 includes agent delivery components (e.g. when second device 900 comprises an implanted and/or external drug delivery pump) that adjust drug or other agent delivery automatically. Alternatively or additionally, system 10 can provide information (e.g. via user interface 330) with recommended drug or other agent delivery changes. Alternatively or additionally, system 10 can be configured to monitor brain activity and/or other physiologic parameters (e.g. monitoring of one or more biomarkers of the patient), to perform dynamic, informed surgery and/or neuromodulation (e.g. stimulation delivered to neuromodulate). System 10 can perform chronotherapy, where forecasts of brain activity are used for dynamic therapeutic interventions, such as behavioral adjustments or medication based on anticipated brain states.

System 10 can be configured to diagnose, prognose, and/or treat **epilepsy,** as described herein. System 10 can perform continuous, long-term, electroencephalography (clEEG) monitoring, and objectivize epileptic brain activity over time. System 10 can provide accurate and objective epilepsy monitoring, including accurately quantifying the number of seizures that occur over a prolonged period. System 10 can capture rare and/or frequent events that are missed in short term monitoring and/or not apparent to the patient or external observers ("subclinical"). Algorithm 50 can enable the quantification of interictal epileptiform activity (IEA) as rates of individual pathological waveforms including spike-waves, polyspikes, rhythmic slowing, bursts of rhythmic activity and high frequency oscillations (HFOs), that are markers of epileptic brain activity. Epilepsy is a cyclical disorder of the brain where seizure risk (the risk of seizure occurrence at any given time) is not uniformly distributed over time. Instead, disease activity fluctuates at multiple timescales including in some patients with circadian patterns or patient-specific multi-day cycles. System 10 can track arousal states and sleep homeostasis (the variable need for sleep) that influence seizure threshold and incidence (i.e. lack of sleep may lower threshold). Characterizing the combinatory influences of endogenous and exogenous risk factors for seizures at the individual level will enable anticipation of seizure risk through personalized probabilistic models employed by algorithm 50. System 10 can compute real-time seizure risk (forecasting) taking all these features into account. Understanding of an individual patient's disease activity at this level of precision allows for several applications. System 10 can perform highly precise objectivization of disease activity for medication titration. System 10 can provide feedback information on fluctuations in disease activity for patient-based decision-making, using for example Bayesian probabilistic estimates of seizure likelihood in the coming minutes, hours, days, months. System 10 provided feedback can include one or more warning signals that can be adjusted either in real time or after analysis, based on the data obtained. System 10 can provide personalized chronotherapy that includes dynamic therapeutic adjustments to variable seizure risk. System 10 can provide closed-loop neuromodulation using direct and/or indirect brain electrical stimulation, and quantifying the stimulation's effect on disease activity. System 10 can provide pre-surgical information on localization of a seizure focus based on recordings of hundreds of seizures, as opposed to a few (e.g. 1-10) seizures on average during inpatient epilepsy workups as currently practiced. System 10 can provide stepwise monitored epilepsy surgery through minimally invasive techniques such as recently developed thermocoagulation or ultrasound ablation. Epilepsy is a brain network disease where the seizure onset area forms the hub of a ramifications of irritable areas. Surgical treatment of this network via brain microlesion as opposed to brain parenchyma resections is currently being tested. Using system 10, the effects of each new brain micro-lesion staggered in time could be monitored over weeks of a stepwise surgical approach, to minimize the risk of damaging eloquent or other essential areas. In some embodiments, system 10 includes a patient activatable switch (e.g. a wearable switch, such as functional element 299 and/or 399) that allows a patient to report a patient-identified epileptic event (e.g. a seizure or other epileptic event). The patient activatable switch can be an accelerometer configured to detect one or more taps (e.g. "dual taps", or at least two taps) to record a seizure or other patient-detected event.

In some embodiments, system 10 is configured to diagnose, prognose, and/or treat a **sleep disorder.** System 10 can enable objective monitoring of disease impact on cerebral and cardio-vascular physiology. Sleep Fragmentation (SF), or the disruption of physiological sleep architecture, is a central feature of common sleep pathologies such as breathing or movement-related sleep disorders. SF is also found in narcolepsy, chronic pain, and ageing. SF is known to have broad impact on heart-rate, blood pressure, metabolism, and the endocrine system. SF constitutes a well-recognized cardio-vascular risk factor and can lead to the development of diabetes. Regarding brain functions, SF leads to cortical dysfunction and impairs cognition including attention and memory. These issues can have dramatic consequences such as accidents and can lead to inability of the patient to work. Similar to its application in epilepsy, system10 provides precise quantitative medical management by documenting sleep quality metrics (e.g. arousal index, slow wave activity, sleep latency, REM sleep latency, etc.). Information related to personal sleep patterns at this level of precision allows for numerous applications. For example, system 10 can provide quantification of sleep quality, such as to titrate pharmacological or respiratory assistive devices (e.g. continuous positive airway pressure - CPAP). System 10 can provide feedback information on fluctuations in sleep pressure (also referred to as "sleep homeostasis") and its repercussion on cortical function. System 10 can provide closed-loop neuromodulation of the cortex to reestablish proper cortical function.

In some embodiments, system 10 is configured to perform **stroke rehabilitation.** System 10 can provide electrical neuromodulation, which has been shown to be of benefit, given sufficient participation in stimulation sessions (e.g. transcranial and/or intracranial stimulation). The always-available neuromodulation provided by system 10 can enhance therapeutic effectiveness due to accessibility to therapy at the patient's own time and place. Real time access to brain signals during rehabilitation exercises provides another benefit. Changes in brain signals accompanying stroke recovery provided by system 10 can inform on and objectify brain healing processes, including processes of neuroplasticity, which are regarded as principal underlying factors of physiological stroke recovery.

In some embodiments, system 10 is configured to provide **pain management** to a patient. For pain management, system 10 can provide always available neurofeedback, and/or stimulation (e.g. transcranial stimulation provided by sensors 160 and/or transcranial, epidural, subdural and/or other stimulation provided by any stimulation element of system 10). In some embodiments, system 10 is configured to manage chronic pain and/or neuropathic pain. In some embodiments, sensors 160 comprise bone-penetrating electrodes (sensors 160' described herein), and system 10 provides motor cortex stimulation for pain management (without the need for a craniotomy). The bone-penetrating sensors 160 can be positioned such that their distal tip is positioned proximate the dura mater. In some pain management configurations, second device 900 comprises a stimulation device (e.g. a spinal cord stimulation device), that receives information from external device 200 or other component of system 10, and the received information is used by second device 900 to provide pain-relief or other therapy to the patient (e.g. in a closed-loop arrangement).

In some embodiments, system 10 is configured to diagnose, prognose, and/or treat a patient in **a coma.** System 10 can be configured to monitor brain activity and neuro-resuscitation. This is particularly indicated in cases were restoration of cerebral function is known to take several days and where recovery can be hindered by neurological complications. These conditions can apply to cerebral traumatic injury and subarachnoid hemorrhage, where seizures are frequent and delayed vascular complications (e.g. vasospasm) frequently occur and can be detected by system 10. Timely detection of these complications enables rapid reactivity and treatment optimization.

In some embodiments, system 10 is configured to diagnose, prognose, and/or treat **neuropsychiatric disorders.** Among psychiatric disorders, depression is a leading cause of disability and a public health priority. For depression and other mood disorder afflicted patients, system 10 can monitor EEG biomarkers including changes in sleep-wake cycle, arousal and/or connectivity. System 10 can also provide stimulation to the brain (e.g. electrical stimulation such as transcranial electrical stimulation delivered by one or more sensors 160 and/or direct cerebral stimulation (e.g. deep brain stimulation, DBS). Obsessive-compulsive disorders, tic disorders and Tourette syndrome are commonly thought of as abnormal reinforcement of loops of network activity that is reflected in repetitive behavior. For patients afflicted with these medical conditions, system 10 can provide neuromodulation (e.g. neuromodulation, as described herein, of frontal networks) as a treatment. Attention and hyperactivity disorders are related to similar abnormal loops of cerebral activity and again, system 10 can provide neuromodulation as a treatment.

In some embodiments, system 10 is configured to diagnose, prognose, and/or treat **neurodegenerative disease.** Alzheimer disease is the leading cause of dementia and a public health problem in developed countries. Recently, abnormal epileptic activity limited to the hippocampus, a deep brain structure involved in memory, has been demonstrated. Importantly, these very focal seizures were shown using depth electrodes and were never seen on the surface of the brain. This observation opens the possibility of using one or more anti-seizure therapeutics to treat Alzheimer disease. In some embodiments, system 10 can include depth electrodes targeting the hippocampus (e.g. functional elements 199 and/or 999 comprising depth electrodes) to monitor brain activity in Alzheimer disease. In these embodiments, system 10 can provide neuromodulation and/or provide information to titrate anti-seizure drugs (and/or other medications) to alleviate memory-related symptoms. In some embodiments, system 10 can be configured to diagnose, prognose, and/or treat other neurodegenerative diseases as well.

In some embodiments, system 10 is configured to diagnose, prognose, and/or treat **demyelinating disease.** Multiple sclerosis (MS) is a chronic disorder characterized by demyelination of white matter tracts, which results in slowed cerebral or spinal cord conduction and consequently neural dysfunction. Initially, MS occurs as relapses and remissions. Continuously monitoring brain connectivity (e.g. inter-hemispheric) by system 10 could enable early detection of relapses.

In some embodiments, system 10 is configured to diagnose, prognose, and/or treat **tinnitus.** For example, system 10 can perform monitoring (e.g. EEG and other brain signal monitoring and/or monitoring from fNIRS sensors), such as monitoring that is performed continuously for long periods of time (e.g. at least 2 weeks, or at least 2 months). Neurofeedback can be provided by system 10, for example continuously and for long periods of time, based on this monitoring. Using this neurofeedback, the patient can learn how to control their pathological auditory cortex activity, at their own pace and place, without any additional burden for them. Such optimized rehabilitation training can facilitate sustained cortical reorganization of the auditory system that then reflects on the reduction of tinnitus' intensity. In some embodiments, system 10 provides a similar approach to treat a wide range of other brain circuit disorders.

In some embodiments, system 10 is configured to diagnose, prognose, and/or treat **dyslexia** (used herein to include dyslexia and other dyscognitive disorders such as dyscalculia, dysphasia, and the like). System 10 can be configured to provide brain stimulation (e.g. transcranial stimulation provided by sensors 160 positioned on the top of the skull or other stimulation as described herein) to treat dyslexia. Dyslexia can be the consequence of abnormal brain rhythms that are normally used to structure language perception and/or production. In some embodiments, system 10 can provide electrical stimulation (e.g. using specific frequency, intensity, and/or duration) to cause the restructuring of a brain circuit that restores normal brain rhythms and thus eliminates or at least reduces dyslexia. In these embodiments, system 10 can provide continuous monitoring (e.g. EEG and/or other brain signal monitoring) of the auditory cortex, and provide electrical stimulation (e.g. closed-loop transcranial or other electrical stimulation of the brain, as described herein) based on the monitoring. The stimulation can be provided on an as-needed basis, and can reorganize brain rhythms to treat dyslexia.

In some embodiments, system 10 monitors brain activity as well as other activity, such as when one or more sensors of system 10 record EEG or other brain activity as well as two or more of: temperature (e.g. skin temperature), pressure, heart rate (e.g. via one or more photoplethysmogram sensors), motor behavior (e.g. a measure of gait, fatigue, and/or falls via one or more accelerometers); and/or speech and/or other verbal output of the patient (e.g. via one or more microphones and/or skull vibration sensors as described herein).

As described herein, system 10 can be **configured to stimulate tissue** of the patient, such as stimulation that is delivered based on the analysis of brain activity and/or other physiologic parameter information recorded by system 10 (e.g. recorded by sensors 160 and/or other sensors of system 10). In some embodiments, sensors 160, functional element 199, functional element 99a, functional element 99b, functional element 999, and/or another component of system 10 is configured as a stimulation element that delivers stimulation (e.g. delivers stimulation energy and/or a stimulating agent) to the patient. In some embodiments, two or more separate stimulation elements of system 10 deliver stimulation to the patient, simultaneously or sequentially. In some embodiments, system 10 comprises one or more stimulation elements that are configured to stimulate the vagal, trigeminal or other cranial or peripheral nerve of the patient. In some embodiments, system 10 comprises one or more stimulation elements that are configured to stimulate the heart of the patient, such as to pace and/or defibrillate the patient's heart. In these embodiments, system 10 can be configured to prevent sudden expected death in epilepsy (SUDEP).

In some embodiments, system 10 is configured to perform **electrical impedance tomography,** where the array of subcutaneously implanted electrodes is used to measure spatial distribution of electrical conductivity and/or impedance of the brain, which can then also be used to form a tomographic image of the brain in respect to said conductivity and/or impedance. Changes in the conductivity and/or impedance can be used by system 10 to monitor disease events and/or progression, for example epileptic seizures of the patient.

In some embodiments, system 10 is configured to deliver **temporal interference (TI) electrical stimulation.** In these embodiments, sensors 160 (e.g. stimulation-providing electrodes positioned on the subcutaneous plane below the skin and above the skull) can deliver stimulation energy (e.g. electrical energy) to brain tissue that is: relatively deep (e.g. deeper than would practically be delivered by a standard transcranial electrical stimulation system); and/or a relatively specific, limited volume of tissue (e.g. a focused energy delivery that is more specific than would practically be delivered by a standard transcranial electrical stimulation system). System 10 can use TI stimulation to stimulate deep tissue of the cortex, such as tissue in a sulcus of the cortex, with or without avoiding stimulating superficial tissue of the cortex. Sensors 160 can deliver TI stimulation that stimulates relatively deep tissue, while avoiding (significantly) stimulating tissue that is more proximal to the sensors 160. In some embodiments, sensors 160 can be configured and arranged to be positioned below the skin and under the skull, and to stimulate brain tissue areas that in current clinical practice are stimulated using a commercially available deep brain stimulation (DBS) device. In some embodiments, two or more electrode-based sensors 160 are positioned above the skull of the patient, and system 10 delivers TI stimulation (e.g. at high amplitude) to stimulate tissue that is at least 10mm, 20mm, and/or 40mm below the surface of the brain (e.g. below the cerebral convexity directly facing the cranial vault), such as to provide one or more stimulation-based therapies as described herein. In some embodiments, stimulated tissue includes, but is not limited to, tissue of the: motor cortex; nucleus accumbens; subthalamic nucleus (STN); and/or globus pallidus interna (e.g. for a Parkinson's Disease patient). Stimulation fields can be generated in a monopolar arrangement, where stimulation current is delivered between one or more electrode-based sensors 160 and a common electrode (e.g. an electrode-portion of housing 101 of implantable device 100, such as common electrode 111 described hereabove). Alternatively or additionally, stimulation fields can be generated in a bipolar or other multipolar arrangement, where stimulation current is delivered between two or more sensors 160. Energy can be delivered "on-demand" (e.g. only when needed), periodically, and/or continuously.

In these TI stimulation embodiments, a first pair of electrodes (e.g. a sensor 160 electrode and common electrode 111 or two sensor 160 electrodes) can generate an electric field at a first frequency (e.g. a frequency of approximately 1.0 kHz or higher that is generally assumed to be too high to interact with neural tissue, such as to stimulate neurons), while a second pair of electrodes (e.g. a sensor 160 electrode and common electrode 111 or two sensor 160 electrodes) generate an electric field at a second, slightly different frequency (e.g. a frequency of approximately 1.01 kHz). The difference in frequencies (e.g. a difference of 10Hz) results in a modulation of the envelope of the alternating fields in a region where the fields interfere, causing a generation of current in relatively deep brain locations as described hereabove. This configuration can be applied to two, three, four, or more pairs of electrodes (e.g. where two or more pairs can share the same electrode), with differences in drive signal frequencies (e.g. drive signals provided by IPU 120) that are small enough to stimulate neural tissue (e.g. on the order of a few Hz to low hundreds of Hz, such as a difference of at least 2Hz and/or at most 500Hz). The resultant interference region (where current is generated) can be selected to occur much deeper in the brain than would be stimulated using conventional transcranial alternating current stimulation (tACS), thus enabling deep brain therapy without the need of a craniotomy and intracranial implants. In some embodiments, recordings made by sensors 160 can be used to deliver TI electrical stimulation, such as to deliver closed-loop therapy comprising two or more interfering electrical fields delivered by multiple sets of sensors 160 based on electrical activity of the brain recorded by sensors 160. In some embodiments, a configuration (e.g. titration) step is performed, in which a series of different electrode-based sensor 160 pairs are chosen to generate the electric fields, and subsequently the pairs that properly interfere and stimulate the desired region of tissue (e.g. brain tissue) are selected to provide a therapeutic benefit for the patient. In some embodiments, a similar titration step is performed to select the best electrode pairs to "steer" the delivered current (and the respective TI stimulation) in a desired manner.

**Referring now to** **Fig. 2****,** a schematic view of another embodiment of a neural interface system including an implantable sensor device and an external processing device is illustrated. System 10 includes implantable device 100 and external device 200, and other components, each of which can be of similar construction and arrangement to those described in reference to Fig. 1 or otherwise herein. Implantable device100 comprises lead assembly 150 which includes multiple leads 151 (six shown). Each lead 151 comprises one or more sensors 160 (2 shown on each lead 151). Sensors 160 can comprise electrodes, fNIRS sensors, and/or other sensors. Leads 151 can be positioned on the skull and under the skin, such as to record brain activity of the patient. Lead assembly 150 is operably connected to an implantable transmitter, ITX 110, via conduit 152, each as shown. ITX 110 can be positioned within housing 101. In some embodiments, implantable device 100 also includes implantable processing unit 120 and/or functional element 199, each of which can be positioned on and/or within housing 101, as shown. In some embodiments, lead assembly 150 comprises one or more functional elements 199, also as shown, such as transducer and/or sensor based functional elements 199.

System 10 further includes external device 200 which can be positioned proximate implantable device 100 (e.g. close to the skin proximate the implantation site of ITX 110) such that a receiver, ETX 210 can receive wireless transmissions of information from ITX 110. In some embodiments, ETX 210 can transfer information and/or power to ITX 110 and/or another component of system 10. External device 200 can also include EPU unit 220, user interface 230, and/or ESA 240, each as described hereabove in reference to Fig. 1. EPU unit 220, user interface 230, and/or ESA 240 can be positioned within a single housing, such as housing 201 shown, and operably connected (e.g. at least electrically connected) to ETX 210 via conduit 212 (e.g. including at least one or more wires). Housing 201 can be attached (e.g. releasably attached) to a belt or other strap, strap 211 shown, for attachment proximate the patient. External device 200 can comprise one or more functional elements, such as functional elements 299a and/or 299b shown attached to strap 211. Functional elements 299a and/or 299b can be configured as described hereabove in reference to Fig. 1. In some embodiments, functional elements 299a and/or 299b comprise a sensor such as an accelerometer and/or gravimetric sensor configured to provide a signal related to patient motion and/or patient position.

System 10 can include controller 300a, a clinician controller, which can take the form of a laptop (as shown), smartphone, tablet, and the like. System 10 can further include controller 300b, a patient controller, which can take the form of a smartphone (as shown), laptop, tablet, and the like. Controllers 300a and/or 300b, as well as external device 200 can transfer information between them, such as information based on information transmitted by implantable device 100 to external device 200. Information transfer can be via a wired or wireless connection. Various information can be uploaded (from external device 200, clinician controller 300a and/or patient controller 300b) to network 600 (e.g. the internet or other computer network), such as to be transferred to server 500. Server 500 can include global processing unit 510 and cloud 550, each as described hereabove in reference to Fig. 1. Global processing unit 510 can comprise algorithm 50, which can include one or more algorithms configured to analyze information (e.g. physiologic information of the patient), such as to perform or enhance a diagnosis, prognosis, and/or treatment of the patient.

Implantable device 100 can be configured to record electrical activity and/or deliver electrical energy, via one or more electrode-based sensors 160, in a monopolar or multipolar (e.g. bipolar) arrangement. In some embodiments, all or a portion of housing 101 comprises a conductive portion, such as common electrode 111 shown, which can function as a return path for recorded signals and/or delivered energy.

**Referring now to** **Figs. 3A-C,** top views of three lead assemblies are illustrated. Various configurations of lead assemblies 150 are shown in Figs. 3A-C, and each can be implanted such that its sensors 160 (e.g. at least electrodes) are positioned in a subcutaneous plane (e.g. on the top surface of the skull of the patient). Lead assembly 150 can be of similar construction and arrangement to lead assembly 150 described hereabove in reference to Fig. 1. In some embodiments, lead assembly 150 comprises one or more leads 151 as described in reference to Fig. 3A, one or more leads 151 as described in reference to Fig. 3B, and/or one or more leads 151 as described in reference to Fig. 3C. Each lead 151 can comprise a substrate including one or more flexible materials as described herein. Each lead 151 can comprise multiple sensors 160 (e.g. multiple electrodes, four shown in each of Figs. 3A-C). Each lead 151 can be attached to a wire, trace, or other electrical conduit, conductors 154. The distal end of each lead comprises a tip, such as reinforced tip 1513 shown and described herebelow in reference to Figs. 8A-B. Lead 151 can comprise a substrate (e.g. a shaft) with a relatively flat geometry, a tubular geometry, or segments with each of these. Each sensor 160 can be positioned such that its exposed surface is relatively continuous with the surface of the lead 151 (e.g. each sensor 160 has a relatively small thickness and/or sensor 160 is embedded within lead 151).

**In** **Fig. 3A****,** lead assembly 150 comprises at least one lead 151 that comprises at least one sensor 160 (four shown). Lead assembly 150 can be constructed and arranged to allow simplified surgical implantation and to conform with the topology of the patient's skull or other implant location. Lead 151 can comprise a substrate with a relatively flat geometry, and can comprise a width of up to 3.5mm and a length of approximately 10cm. Each lead 151 can comprise multiple electrode-based sensors 160 with a length of approximately 5mm, and each sensor 160 can be positioned on the substrate of lead 151 with an approximately 25mm center-to-center separation distance from a neighboring sensor 160. Each sensor 160 can be attached to a different wire or other electrical conduit, conductors 154, four shown. Each conductor 154 can be operably attached to ITX 110. Lead 151 can comprise a reinforced tip, reinforced tip 1513 shown and described herebelow in reference to Figs. 8A-B.

**In** **Fig. 3B****,** lead assembly 150 comprises at least one lead 151 that comprises at least one sensor 160 (four shown). Lead assembly 150 can be constructed and arranged to allow simplified surgical implantation and/or explantation, such as with the minimum tools required. Leads 151 can comprise a substrate with a tubular shape, such as a tube with a diameter of approximately 1.8mm and/or a length of approximately 10cm. Each sensor 160 (e.g. 4 or 5 electrodes) can comprise a ring shape, and can be mono-directional and/or multi-directional (e.g. with a commuter to steer directionality towards the patient's brain or other target site). Each lead 151 can comprise multiple electrode-based sensors 160, 4 shown. Each sensor 160 can be attached to a different wire or other electrical conduit, conductors 154, not shown but as described herein. Each conductor 154 can be operably attached to ITX 110. Lead 151 can comprise a reinforced tip, reinforced tip 1513 shown and described herebelow in reference to Figs. 8A-B

**In** **Fig. 3C****,** lead assembly 150 comprises at least one lead 151 that comprises at least one sensor 160 (four shown). Lead assembly 150 can be constructed and arranged to provide enhanced signal-to-noise ratio and reduced artifact contamination. Leads 151 can comprise a substrate with a width of up to 10mm, such as to support one or more sensors 160 that comprise concentric ring electrodes (e.g. up to 10 concentric ring electrodes). For example, a sensor 160 can comprise multiple ring electrodes that allow system 10 to incorporate (e.g. in algorithm 50) an approximated Laplacian derivation of a measured electrical potential, improving signal quality over conventional single disc electrodes. In some embodiments, adverse effects to signal quality due to muscle-induced artifacts can be reduced (e.g. artifacts from the sub-temporal muscle or other muscle proximate a sensor 160). A center disk electrode can be surrounded by one or more concentric ring electrodes, as shown. In some embodiments, a center disk electrode is surrounded by between 2 and 10 concentric ring electrodes, such as between 2 and 5 concentric ring electrodes. In some embodiments, the center disk electrode comprises a diameter of approximately 2.7mm. In some embodiments, the outermost ring electrode comprises a diameter of at least 5mm, such as at least 10mm. Each sensor 160 can be attached to a different wire or other electrical conduit, conductors 154, not shown but as described herein. Each conductor 154 can be operably attached to ITX 110. Lead 151 can comprise a reinforced tip, reinforced tip 1513 shown and described herebelow in reference to Figs. 8A-B.

**Referring now to** **Figs. 4A-C,** various views of a lead assembly including a bone penetrating sensor is illustrated. In Fig. 4A, a top view of lead assembly 150 is shown, including lead 151. In Fig. 4B, a side-sectional view of lead assembly 150 is shown, including lead 151 and a bone-penetrating sensor, sensor 160'. In Fig. 4C, a side sectional view of lead assembly 150 is shown, in which sensor 160' has been inserted through lead 151. Lead assembly 150 and/or sensor 160' can be of similar construction and arrangement to the similar components described hereabove in reference to Fig. 1. Sensor 160' can include shaft 161, and in some embodiments, shaft 161 comprises threads 164 as shown (e.g. nonconducting threads configured to frictionally engage bone or other tissue). Sensor 160' of Fig. 4B includes active portion 163 positioned on the distal end of shaft 161, such as a conductor when sensor 160' comprises an electrode-based sensor. Sensor 160' can comprise a flange, cap 162 shown on the proximal end of shaft 161, which can include an engagement portion for a tool such as a screwdriver, as described herebelow in reference to Fig. 4D.

Shaft 161 can be positioned within (e.g. screwed into a pilot hole made with a tool 800 comprising a surgical drill) the patient's skull, such that active portion 163 is positioned within the skull (e.g. flush, near-flush, and/or above the inner table of the skull), or into the intracranial space (e.g. above and/or into the patient's brain, such as at a positioning making contact with the dura without penetrating the dura). Shaft 161 can comprise a non-conductive material, while active portion 163 comprises a conductive material, such as to limit brain signal recording to only the tip portion of sensor 160'. In some embodiments, one or more portions of cap 162 (e.g. a bottom portion) includes a conductive portion which is electrically attached to active portion 163 by a conductive filament, not shown but positioned on and/or within shaft 161. In these embodiments, when sensor 160' is positioned thru lead 151 as shown in Fig. 4C, the conducting portion of cap 162 can be electrically connected to conductive portion 153 of lead 151, with conductive portion 153 electrically attached to conductor 154 (e.g. a wire or conductive trace). Conductive portion 153 comprises a receiving hole surrounded by a conductive element (e.g. a conductive ring). Conductor 154 travels to an electrically connecting portion of lead assembly 150, such as to connect to ITX 110 as described hereabove in reference to Fig. 1.

In some embodiments, one or more sensors 160' of lead assembly 150 comprise fNIRS sensors, as described hereabove. In these embodiments, the one or more fNIRS-based sensors 160' can be positioned thru the skull, such as to measure cerebral hemodynamics with enhanced resolution and/or signal-to-noise ratio as compared to a scalp-mounted fNIRS sensor.

Sensors 160' can be insertable into lead 151 at conductive portion 153, and/or pre-attached (e.g. rotatably attached) to conductive portion 153 of lead 151. In some embodiments, each sensor 160' can be inserted into and/or engaged with bone (e.g. the skull) via an incision in the skin directly above the insertion location in the bone, such as via a standard screwdriver tool. Alternatively, each sensor 160' can be inserted into and/or engaged with bone (e.g. the skull) via an incision in the skin offset from the insertion location into the bone (e.g. the same incision where all or a part of lead 151 is inserted), such as via a right-angle arranged tool, such as tool 820a shown in Fig. 4D.

**Referring additionally to** **Fig. 4D****,** a not-to-scale view of a right-angle tool inserting an intra-bone sensor into a patient skull is illustrated. Right-angle tool 820a can comprise a handle 821, a shaft 822, and rotating assembly 825 as shown. Rotating assembly 825 can be configured to be secured and rotatably engaged to a sensor 160'. Rotating assembly 825 can be configured as a ratcheted rotator, and/or a motor-driven rotator, such as might be activated by an operator control 824 positioned on handle 821. With a sensor 160' attached, a clinician holding handle 821, can advance rotating assembly 825 and sensor 160' through an incision (e.g. an incision near the apex of the patient's head, such as an incision less than 2cm), through subcutaneous tissue (e.g. previously dissected tissue) to a location remote from the apex incision that is proximate a bone site for implantation of sensor 160' (e.g. a location in the patient's skull). Subsequently, threads 164 of sensor 160' can engage the bone (e.g. via a self-drilling tip or into a previously drilled hole), and advance into the bone (e.g. via rotation applied by rotating assembly 825), as shown in Fig. 4D. These bone-engaging steps can be repeated for multiple sensors 160' of a single lead 151, and for other sensors 160' of other leads 151.

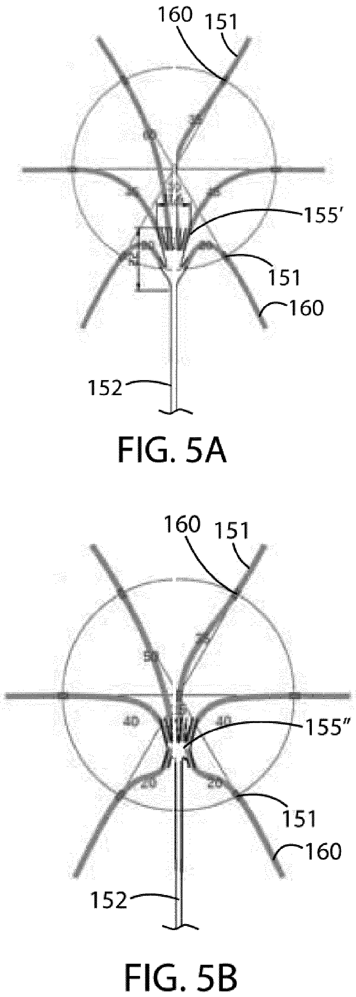

**Referring now to** **Figs. 5A-B,** a top view of two lead assemblies is illustrated. Lead assemblies 150 of Figs. 5A-B each include multiple leads 151. Each lead assembly 150 can be of similar construction to lead assembly 150 described hereabove in reference to Figs. 1, 3A-C, and/or 4A-C. Each lead assembly 150 can comprise similar or dissimilar leads 151. Lead assembly 150 includes conduit 152, which includes one or more conductors which operably connect each lead 151 (e.g. each sensor 160) to ITX 110. In some embodiments, conduit 152 connects ITX 110 to one or more other sensors, such as one or more sensor based functional elements 199 as described hereabove in reference to Fig. 1.

Lead assemblies 150 can be implanted with the multiple leads 151 in the star-shaped geometry shown, such as to obtain broad coverage of a tissue site such as the patient's brain when lead assembly 150 is positioned under the skin, on top of the skull of the patient. In this configuration, global brain activity can be monitored (e.g. when sensors 160 comprise electrodes and/or other sensors for recording electrical activity of the patient's brain). Lead assemblies 150 each comprise multiple leads 151 which can comprise flexible material allowing folding of leads 151 into a relatively linear arrangement (e.g. in a tube-shaped geometry to support insertion of multiple leads 151 through a relatively small incision in the patient's skin, such as using a tube-shaped introducer tool 810). Each lead assembly 150 comprises a central hub, hub 155, from which the multiple leads 151 extend. Leads 151 can be arranged with relatively equidistant spacing as shown, or otherwise (e.g. to provide denser coverage of one area versus another). For example, all leads 151 can be positioned over a single brain hemisphere or a single lobe). In some embodiments, hub 155 arranges leads 151 in the "fork-like" configuration, such as hub 155' shown in Fig. 5A. Alternatively, hub 155 can arrange leads 151 in a "gear-switch" arrangement, such as hub 155" shown in Fig. 5B. In some embodiments, hub 155 arranges leads 155 in a "star geometry", such as is described herebelow in reference to Fig. 6. In some embodiments, hub 155 can comprise an electronics assembly, such as an electronics assembly that applies signal processing to signals recorded by sensors 160 (e.g. digitizing, multiplexing, electronic switching, amplifying, noise reducing, and the like). In some embodiments, hub 155 comprises an electronics assembly that reduces the number of wires needed in conduit 152 (e.g. including switches or multiplexing functions that avoid conduit 152 having an individual wire, or individual wire pairs, for each sensor 160).

**Referring now to** **Fig. 6****,** a perspective view of a patient's head into which a lead assembly and implantable transmitter have been implanted is illustrated. Implantable device 100 is configured to be implanted in a minimally invasive procedure, as described herein, without the need for a craniotomy. Implantable device 100 can be implanted without the need for expensive imaging equipment, nor the need for an expensive operating room. Lead assembly 150 can be inserted under the patient's skin, above the patient's skull, as shown in Fig. 6. For example, lead assembly 150, comprising multiple leads 151 (6 shown) each comprising at least one sensor 160, can be surgically implanted between the skull periosteum and the scale galea aponeurosis, and/or other subcutaneous surgical planes. In some embodiments, in order to implant implantable device 100 (e.g. including lead assembly 150 and ITX 110), two incisions are made, a retroauricular (behind-the-ear) incision, incision A shown, and an incision at the vertex of the head, incision B shown. Conduit 152 of lead assembly 150 is tunneled between incision A and incision B, and ITX 110 is implanted via incision A. In some embodiments, lead assembly 150 is pre-attached (via conduit 152) to ITX 110, and leads 151 are inserted through incision A and tunneled to and exiting incision B. In some embodiments, lead assembly 150 is attachable to ITX 110 (e.g. an attachment made by the implanting clinician), and conduit 152 can be tunneled from incision A to incision B or from incision B to incision A. Leads 151 of lead assembly 150 are passed through incision B (e.g. after having been tunneled from incision A to incision B or not), and each lead 151 tunneled to a desired location over the skull (e.g. into the star pattern shown in Fig. 6). In some embodiments, a chest incision can be made, as an alternative to incision A or in addition to incision A (for example when ITX 110 is implanted in the chest).

Incision B can be positioned under the patient's hair, and can comprise an incision with a length of no more than 6cm, such as no more than 5cm, no more than 4cm, and/or no more than 3cm. Incision A can comprise an incision with a length of no more than 6cm, such as no more than 5cm, no more than 4cm, and/or no more than 3cm. In some embodiments, incision A is longer than incision B, such as when implantable device 100 is inserted behind the patient's ear through incision A. In some embodiments, incision A is of similar length to incision B, such as when implantable device 100 is inserted through a different incision in the patient's chest, and incision A is used as a midpoint of tunneling leads assembly 150 from the chest incision, to incision A, and then to incision B.

In some embodiments, a first step can include marking or at least determining the locations for incisions A and B. Another step includes folding leads 151 into a linear arrangement, positioning the leads 151 into a narrow tube (e.g. an introducer tool 810 comprising a narrow tube), inserting the leads 151 and introducer tool 810 into incision A, and subcutaneously tunneling the leads 151 (including the introducer tool 810) towards and exiting incision B. In some embodiments, a second introducer tool 810 comprises a trocar and/or raspatory tool used to assist in the tunneling (e.g. to create a first tunnel through which a second tunneling tool attached to lead 151 is advanced). A subsequent step includes removing the leads 151 from the tube of introducer tool 810, and inserting each lead 151 back into incision B to a location above the scalp, such as to collectively orient the multiple leads 151 in a desired coverage pattern, such as the star-shaped pattern shown in Fig. 6. Each lead can be inserted with a tool 800 comprising a introducer tool 810. Introducer tool 810 is configured to detachably engage a distal portion of lead 151, such as is described herebelow in reference to Figs. 8A-B. In some embodiments, as described above, tool 800 can further comprise a first introducer tool 810 used to create a first tunnel through which a second introducer tool 810 (attached to one or more leads 151) is to be subsequently inserted. After each lead 151 is inserted, the introducer tool 810 is detached and removed (leaving the lead 151 in place), and subsequently attached to another lead 151 to be inserted. These steps are continued until all leads 151 are implanted. In some embodiments, during retraction of introducer tool 810, the clinician applies a force, through the scalp, to a distal portion of lead 151 (e.g. a tip that extends beyond introducer tool 810), to reduce migration of lead 151 during introducer tool 810 removal.

In some embodiments, an optional step includes securing the lead 151, such as via bone screws and/or sutures. Alternatively or additionally, one or more sensors 160 can be inserted through or at least into the skull of the patient. Methods of implanting skull-inserted sensors 160 is described hereabove in reference to Fig. 4D.

In another step, ITX 110 is placed through incision A under the skin (e.g. behind the ear and/or in the chest of the patient).

While the method described hereabove in reference to Fig. 6 includes a maximum of two incisions (incisions A and B shown) to implant lead assembly 150, in some embodiments, an alternative or potentially more desirable method can include three, four, or more incisions (e.g. three or more small incisions less than 4cm, 3cm or 2cm), such as when two, three or more incisions are used to tunnel one (e.g. each) of multiple leads 151 through tissue to achieve a target implantation geometry (e.g. to a target distal location in a particular linear or curvilinear geometry).

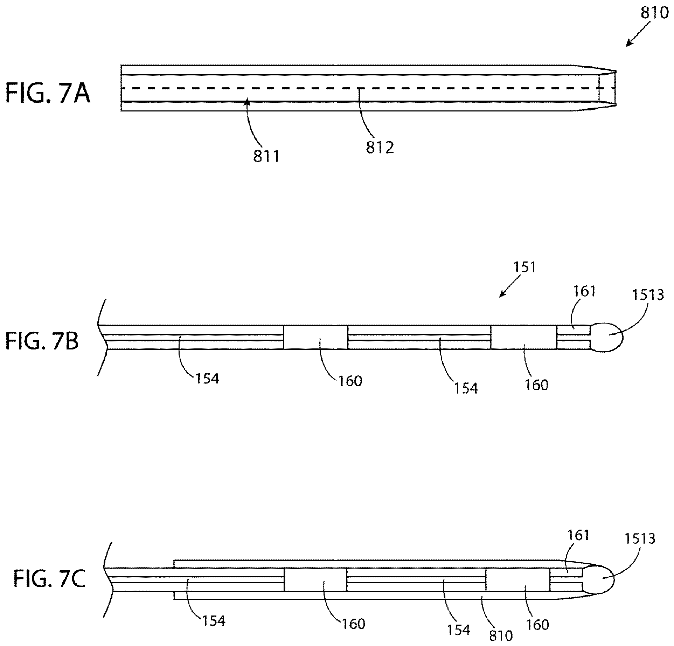

**Referring now to** **Figs. 7A-C,** side sectional views of an inserter tool, a lead, and an inserter tool engaged with the lead, respectively, are illustrated. Introducer tool 810 of Figs. 7A-C comprises a lumen, lumen 811, sized to slidingly receive one or more leads 151. In some embodiments, tool 810 comprises a slit, slit 812 shown, along its length, such that lead 151 can laterally enter lumen 811. Alternatively or additionally, introducer tool 810 can be configured to radially expand, such as to allow a bulbous portion of lead 151 (e.g. bulbous portion of tip 1513 shown) to slide through a lumen 811 that is resiliently biased at a diameter smaller than the diameter of tip 1513 (e.g. to insert lead 151 through lumen 811 and/or to extract lead 151 from lumen 811). In some embodiments, a second introducer tool 810 is included, such as to create a tunnel in tissue through which introducer tool 810 of Figs. 7A-C is to be passed (e.g. when lead 151 is inserted within lumen 811). In some embodiments, tool 810 with an inserted lead 151 is passed through an incision, and advanced under the skin of the patient (e.g. through a tunnel in tissue above the skull of the patient) to a target implant location. Subsequently tool 810 is removed, leaving lead 151 in place. During tool 810 removal, the implanting clinician can apply a force, through the skin, to the tip of lead 151, prevent migration of lead 151 during tool 810 removal. Tool 810 can be removed by laterally disengaging from lead 151 through slit 812 (e.g. when lead 151 is pre-attached to a hub or other connector, as described herein, which is bigger than the diameter of lumen 811). Alternatively, lead 151 can simply exit through the proximal end of tool 810 (e.g. when the proximal end of lead 151 is not attached to a hub or other connector).

**Referring now to** **Figs. 8A-C,** leads with reinforcing members are illustrated. Each lead 151 comprises a sensor 160 (e.g. an electrode), which is electrically connected to conductors 154 (e.g. a bundle of one or more wires, such as when a single or pair of wires connect to each sensor 160). Conductors 154 travel proximally within lead 151, such as to electrically connect to ITX 110. In Fig. 8A, lead 151 comprises one or more reinforcing filaments 1511 (one shown). Reinforcing filament 1511 can comprise one or metal and/or plastic flexible filaments that are positioned on and/or within the tubular substrate of lead 151. In some embodiments, reinforcing filament 1511 comprises suture. In Fig. 8C, lead 151 comprises a reinforcing filament 1511 that travels from a proximal portion of lead 151, exits the distal end of lead 151, re-enters lead 151 (forming a loop as shown), and travels to the proximal portion of lead 151 (e.g. providing twice the load capability as compared to a single filament). In some embodiments, filament 1511 forms a loop within and near the distal end of lead 151 (e.g. without exiting the distal end of lead 151). In Fig. 8C, lead 151 comprises a reinforcing mesh, 1512. Reinforcing mesh 1512 can comprise a metal and/or plastic (e.g. polypropylene) flexible mesh that is positioned on and/or within the tubular substrate of lead 151. Each sensor 160 can be placed above or below mesh 1512. In some embodiments, one or more segments of a lead 151 comprises at least one reinforcing filament 1511 and one or more segments of lead 151 comprises at least one reinforcing mesh 1512.

Lead 151 of Figs. 8A and 8C each include a reinforced tip, tip 1513, which can be configured to be releasably engaged to a tunneling tool, introducer tool 810 (e.g. forceps or other introducer tool 810 described herein) to tunnel lead 151 through tissue (e.g. subcutaneous tissue between the scalp and the patient's skull). Lead 151 of Fig. 8B can also include a reinforced tip, not shown but such as tip 1513 described herein. Reinforcing filament 1511, reinforcing mesh 1512, and reinforcing tip 1513 comprise materials (e.g. plastic and/or metal reinforcing materials) and a construction configured to withstand forces (e.g. stretching, compression, twisting, and/or abrasion) incurred during implantation (e.g. engagement with one or more tunneling tools 800) and/or during explantation (e.g. engagement with one or more removal tools 800), as well as forces encountered during daily life, each as described herein). In some embodiments, reinforcing filament 1511, reinforcing mesh 1512, and/or reinforcing tip 1513 comprise a material that is of a higher durometer than the material of the substrate (e.g. shaft) of lead 151.

In some embodiments, a lead 151 can comprise attachment element 156, such as an aperture (as shown in Figs. 8A and 8C), a loop (as shown in Fig. 8B) or other feature configured to be releasably engaged by a tool, such as introducer tool 810 configured to engage lead 151, advance lead 151 through tissue, and subsequently release lead 151. In some embodiments, attachment element 156 comprises a magnet or magnetic material, and introducer tool 810 comprises a corresponding magnetic material and/or magnet.

**Referring now to** **Fig. 9****,** a top view of a lead assembly comprising a staggered arrangement of leads is illustrated. Lead assembly 150 can comprise multiple leads 151 which extend from conduit 152 in a staggered arrangement (e.g. the staggered arrangement shown), such as to facilitate volumetric efficiency when in a folded arrangement (e.g. for insertion into introducer tool 810) for tunneling through tissue. Each lead can include one or more sensors 160 (e.g. sensors 160a-c shown), each of which can attach to a conductor 154 (e.g. conductors 154a-c, respectively, which are part of a wire bundle, conductor 154). Leads 151 can be resiliently biased with a takeoff angle, angle Θ shown, such as an angle of at least 5°, such as a takeoff angle Θ between 20° and 45°. Each lead can depart from conduit 152 with a separation distance D_{S} of at least 5mm, such as a separation distance D_{S} between 5mm and 10mm. In these embodiments, introducer tool 810 can comprise a tube-like geometry with a diameter of no more than 6mm (e.g. a tube with an inner diameter sized to accept up to four, five, and/or six leads 151).

**Referring now to** **Fig. 10****,** a perspective view of the distal portion of a lead comprising at least one tubular sensor is illustrated. Lead 151 of Fig. 10 includes a sensor 160 comprising a tubular electrode (also referred to as "ring electrode") positioned about a tubular substrate of lead 151. In some embodiments, sensor 160 comprises a top surface that is relatively flush with the adjacent substrate surfaces of lead 151.

In some embodiments, lead 151 comprises one or more markers, such as when a marker is positioned proximate or otherwise relative to one, two or all sensors 160 of that lead 151. In Fig. 10, lead 151 comprises marker 157, such as a radiopaque marker (e.g. visualizable by a fluoroscope or X-ray based tool 800), an ultrasonic marker (e.g. visualizable by an ultrasound imaging device), a magnetic marker (e.g. visualizable by a magnetic field detection device), and/or an infrared diode (e.g. visualizable by an infrared camera based tool 800). An imaging based tool 800 comprising an imaging device can be used to provide an image of marker 157, to assist in locating the referentially positioned sensor 160.

**Referring now to** **Fig. 11****,** a perspective view of the distal portion of a lead comprising multiple circumferentially placed sensors is illustrated. Sensors 160a-d comprise four facet electrodes that are circumferentially positioned about a lead 151, each sensor 160 individually connected to a separate conductive filament (e.g. a wire), conductors 154a-d shown, each of which traveling within lead 151 to operably attach to ITX 110 (e.g. via a pre-attached and/or operator attached configuration). While the embodiment shown in Fig. 11 shows four circumferentially placed sensors 160a-d, numerous quantities of sensors (e.g. electrodes) can be included, such as between two and fifteen circumferentially placed sensors 160. Each facet electrode sensor 160 can circumferentially span less than 360°, such as no more than 180° (e.g. when lead 151 comprises one to two electrodes), no more than 120° (e.g. when lead 151 comprises one to three electrodes), no more than 90° (e.g. when lead 151 comprises one to four electrodes), or no more than 72° (e.g. when lead 151 comprises one to five electrodes). In the configuration shown in Fig. 11, one or two of facet electrode sensors 160a-d can be selected for recording electrical activity of the brain, and/or for delivering stimulation energy to the brain, such as when the one or two sensors 160a-d that are selected are facing toward the patient's brain. In some embodiments, two or more electrode sensors 160a-d can be selected as differential inputs to the IPU 120, such as when one or more sensors 160a-d faces the patient's brain and one or more sensors 160a-d faces away from the patient's brain, for reducing electrical noise not originating from brain activity. In some embodiments, lead 151 comprises one or more wing-like projections or other stabilizing projection, projections 1514 shown, that reduce rotation of lead 151 after implantation.

After implantation, sensors 160a-d can be individually tested (e.g. automatically, semiautomatically, and/or manually) to determine which one or more sensors 160 provided the best signal (e.g. best brain activity signal), and/or provided the best stimulation, typically the one or more sensors 160 oriented toward the target site of interest (e.g. the brain). This optimal sensor selection process can be performed using one or more predetermined methods performed by algorithm 50. In some embodiments, one or more non-optimal sensors 160 (e.g. sensors 160 positioned away from the brain or other target site) provide signals used by system 10 to perform a noise reduction operation. For example, artifacts can be identified, through analysis of signals recorded by one or more non-optimal sensors 160, and effects of these artifacts removed or at least reduced from the signals provided by the optimal sensors 160. In some embodiments, algorithm 50 uses a blind-source separation methodology to reduce the presence of the artifacts.

**Referring now to** **Figs. 12A-B,** sectional anatomical views of a lead comprising depth-electrodes being inserted into a patient's brain are illustrated. System 10 includes an implantable lead assembly 150' that includes one or more tissue insertable leads 151' (one lead 151' shown). Each lead 151' comprises a guide element 1551 (shown in Fig. 12A), a connector 1552 (shown in Fig. 12B), a conduit 1553, a shaft 1554, and one or more sensors 160' (three shown in Figs. 12A-B). Shaft 1554 is configured for insertion into tissue, such as via a hole in the skull of the patient and into brain tissue as shown in Fig. 12. System 10 can include two tools for inserting shaft 1554, guide element 1551 and inserter tool 1555. Guide element 1551 and/or inserter tool 1555 can comprise disposable tools (e.g. tools used to insert one or more leads 151' in a single clinical procedure. Alternatively, guide element 1551 and/or inserter tool 1555 can comprise reusable tools (e.g. tools configured to be re-sterilized and used to insert multiple leads 151' in multiple clinical procedure). Guide element 1551 is positioned in the skull (e.g. engaged with the skull via threads), and used to guide shaft 1554 as it passes through the tissue (e.g. as advanced by applying a pushing force to shaft 1554 using inserter tool 1555). After insertion, lead 151' can be attached to test tool 830, described hereabove in reference to Fig. 1, via conduit 1553. Conduit 1553 can comprise a conduit including one or more wires that are electrically attached to the one or more sensors 160', such as via mating electrical contacts provided by and between guide element 1551 and shaft 1554. The electrical contacts of shaft 1554 attach to wires (of shaft 1554) that electrically connect to sensors 160'. Test tool 830 can be configured to test recordings made by one or more sensors 160' and/or deliver stimulation energy (e.g. electrical energy) to one or more stimulation-enabled sensors 160', such as to confirm proper placement of the one or more sensors 160'. An optimization procedure can be performed (e.g. a titration procedure, a calibration procedure, and/or other parameter-adjusting procedure) in which shaft 1554 is (further) advanced and/or retracted, and comparisons in recording and/or (response to) stimulation made to optimize placement of sensors 160' (e.g. optimize shaft 1554 insertion depth). In some embodiments, test tool 830 can comprise processing unit 120 and/or another portion of implantable device 100 (e.g. to record and/or stimulate in a titration and/or calibration procedure of each lead 151').

Once lead 151' is in place, inserter tool 1555 can be removed (e.g. and discarded and/or used to insert an additional lead 151'). Guide element 1551 can also be removed, and connector 1552, with conduit 1556 attached, can be operably attached to the proximal end of shaft 1554 as shown in Fig. 12B. Conduit 1556 comprises wires that electrically attach to electrical contacts of connector 1552. Similar to guide element 1551, electrical contacts of connector 1552 provide an electrical connection to sensors 160' via mating contacts of shaft 1554 and the wires of shaft 1554 that electrically connect to sensors 160'. The opposite end of conduit 1556 can be attachable, or pre-attached, to hub 155'. Hub 155' can be secured to the skull of the patient, such as via one or more bone screws. Hub 155' can be attached to ITX 110 as shown, such as via a pre-attached or attachable connection, via conduit 152' (e.g. a conduit comprising wires). Once electrically connected, as described hereabove, ITX 110 can record signals made by one or more sensors 160' and/or deliver stimulation (e.g. an agent and/or stimulation energy such as electrical energy) to sensors 160'.

The above-described embodiments should be understood to serve only as illustrative examples; further embodiments are envisaged. Furthermore, equivalents and modifications not described above may also be employed without departing from the scope of the invention, which is defined in the accompanying claims.

## Claims

1. A neural interface system (10) for a patient comprising:
an implantable sensor device (100) comprising;
an implantable lead assembly (150) for implantation above the skull and below the skin of the patient, and for recording physiologic parameter information of the patient, and
an implantable transmitter (110) for receiving the physiologic parameter information from the implantable lead assembly and for transmitting patient data that is based on the physiologic parameter information;
a conduit (152) which connects the implantable lead assembly to the implantable transmitter, the conduit comprising a central conductor (154); and
an external processing device (200) for receiving the patient data from the implantable transmitter,
wherein the implantable lead assembly comprises multiple electrodes (160), and multiple flexible leads (151) extending from the conduit at angles between 5-45°, in a staggered foldable arrangement from alternating sides of a central axis of the conduit with an axial separation distance of at least 5 mm from each other, each lead comprising at least one electrode and being 5 cm to 15 cm long.

2. The system according to claim 1, wherein the leads are configured to be tunneled under the skin.

3. The system according to any of claims 1 or 2, wherein the electrodes comprise two or more facet electrodes that span less than 180 degrees of a circumferential segment, the two or more facet electrodes being individually selectable and the two or more facet electrodes comprising a first facet electrode for recording the physiologic parameters and a second facet electrode used for noise suppression, the second facet electrode being configured to be oriented away from the skull of the patient after an implantation.

4. The system according to any of claims 1 or 2, wherein the electrodes comprise at least one concentric ring electrode surrounding a central electrode, the implantable lead assembly comprising at least one electrode comprising a shielded portion.

5. The system according to any of the preceding claims, each lead comprises between three and ten electrodes, the multiple leads are configured to be folded into a single tube geometry.

6. The system according to any of the preceding claims, wherein at least a portion of the implantable sensor device is biodegradable, said biodegradable portion comprising at least a portion of a component selected from the group consisting of: a lead or other conduit of the implantable lead assembly; an electrode of the implantable lead; a shaft of the implantable lead; a stimulation element; and combinations thereof.

7. The system according to any of the preceding claims, wherein each lead comprises one or more axial reinforcing elements, each axial reinforcing element comprising a reinforcing filament, said reinforcing filament comprising materials and a construction configured to withstand forces incurred during engagement with one or more tunneling tools and/or during engagement with one or more removal tools.

8. The system according to any of the preceding claims, wherein the implantable lead assembly comprises at least one sensor, the system further comprising a visualizable marker positioned relative to the at least one sensor, the visualizable marker comprising an infrared diode or a marker selected from the group consisting of: radiopaque marker; ultrasound marker; magnetic marker; and combinations thereof.

9. The system according to any one of the preceding claims, wherein the system further comprises at least one intracranial sensor operably connected to the implantable transmitter, the at least one intracranial sensor comprising epidural, subdural, and/or depth electrodes.

10. The system according to any one of the preceding claims, wherein a lead of the implantable lead assembly comprises a substrate with a relatively flat geometry, a width of up to 3.5mm and a length of approximately 10cm, said lead further comprising multiple electrode-based sensors with a length of approximately 5mm, each sensor being configured to be positioned on the substrate of the lead with an approximately 25mm center-to-center separation distance from a neighboring sensor.

11. The system according to any of the preceding claims, wherein said system is configured to stimulate brain tissue to achieve always available neuromodulation.

12. The system according to any one of the preceding claims, wherein the system includes one or more tools (800), said one or more tools comprising an introducer tool (810) configured to tunnel one or more leads of the lead assembly under a skin of the patient.

13. The system according to any one of the preceding claims, wherein it is configured to provide self-administered, always available, neurofeedback.

14. The system according to any one of the preceding claims, wherein one or more sensors of the implantable lead assembly comprises one or more functional near infrared spectroscopy, fNIRS, sensors and/or at least one foramen ovale electrode.

## Patentansprüche

1. Neuralschnittstellensystem (10) für einen Patienten, umfassend:
eine implantierbare Sensorvorrichtung (100), die Folgendes umfasst;
eine implantierbare Aderbaugruppe (150) zur Implantation oberhalb des Schädels und unterhalb der Haut des Patienten und zur Aufzeichnung physiologischer Parameterinformationen des Patienten und
einen implantierbaren Sender (110) zum Empfangen der physiologischen Parameterinformationen von der implantierbaren Aderbaugruppe und zum Übertragen von Patientendaten, die auf den physiologischen Parameterinformationen basieren;
eine Leitung (152), die die implantierbare Aderbaugruppe mit dem implantierbaren Sender verbindet, wobei die Leitung einen zentralen Leiter (154) umfasst; und
eine externe Verarbeitungsvorrichtung (200) zum Empfangen der Patientendaten von dem implantierbaren Sender,
wobei die implantierbare Aderbaugruppe mehrere Elektroden (160) und mehrere sich von der Leitung in Winkeln zwischen 5-45° erstreckende flexible Adern (151) in einer versetzten faltbaren Anordnung von abwechselnden Seiten einer Mittelachse der Leitung mit einem axialen Abstand von mindestens 5 mm voneinander umfasst, wobei jede Ader mindestens eine Elektrode umfasst und 5 cm bis 15 cm lang ist.

2. System nach Anspruch 1, wobei die Adern dazu konfiguriert sind, unter die Haut getunnelt zu werden.

3. System nach einem der Ansprüche 1 oder 2, wobei die Elektroden zwei oder mehr Facettenelektroden umfassen, die sich über weniger als 180 Grad eines Umfangssegments erstrecken, wobei die zwei oder mehr Facettenelektroden einzeln auswählbar sind und die zwei oder mehr Facettenelektroden eine erste Facettenelektrode zum Aufzeichnen der physiologischen Parameter und eine zweite Facettenelektrode, die zur Rauschunterdrückung verwendet wird, umfassen, wobei die zweite Facettenelektrode dazu konfiguriert ist, nach einer Implantation vom Schädel des Patienten weg ausgerichtet zu sein.

4. System nach einem der Ansprüche 1 oder 2,
wobei die Elektroden mindestens eine konzentrische Ringelektrode umfassen, die eine zentrale Elektrode umgibt, wobei die implantierbare Aderbaugruppe mindestens eine Elektrode umfasst, die einen abgeschirmten Abschnitt umfasst.

5. System nach einem der vorhergehenden Ansprüche, wobei jede Ader zwischen drei und zehn Elektroden umfasst, wobei die mehreren Adern dazu konfiguriert sind, in eine einzelne Rohrgeometrie gefaltet zu werden.

6. System nach einem der vorhergehenden Ansprüche, wobei mindestens ein Abschnitt der implantierbaren Sensorvorrichtung biologisch abbaubar ist, wobei der biologisch abbaubare Abschnitt mindestens einen Abschnitt einer Komponente umfasst, die aus der Gruppe ausgewählt ist, die aus Folgendem besteht: einer Ader oder einer anderen Leitung der implantierbaren Aderbaugruppe; einer Elektrode der implantierbaren Ader; einem Schaft der implantierbaren Ader; einem Stimulationselement; und Kombinationen davon.

7. System nach einem der vorhergehenden Ansprüche, wobei jede Ader ein oder mehrere axiale Verstärkungselemente umfasst, wobei jedes axiale Verstärkungselement ein Verstärkungsfilament umfasst, wobei das Verstärkungsfilament Materialien und eine Konstruktion umfasst, die dazu konfiguriert ist, Kräften standzuhalten, die während des Eingriffs mit einem oder mehreren Tunnelwerkzeugen und/oder während des Eingriffs mit einem oder mehreren Entfernungswerkzeugen auftreten.

8. System nach einem der vorhergehenden Ansprüche, wobei die implantierbare Aderbaugruppe mindestens einen Sensor umfasst, wobei das System ferner eine visualisierbare Markierung umfasst, die relativ zu dem mindestens einen Sensor positioniert ist, wobei die visualisierbare Markierung eine Infrarotdiode oder eine Markierung umfasst, die aus der Gruppe ausgewählt ist, die aus Folgendem besteht: strahlungsundurchlässige Markierung; Ultraschallmarkierung; magnetische Markierung; und Kombinationen davon.

9. System nach einem der vorhergehenden Ansprüche, wobei das System ferner mindestens einen intrakraniellen Sensor umfasst, der funktionsfähig mit dem implantierbaren Sender verbunden ist, wobei der mindestens eine intrakranielle Sensor epidurale, subdurale und/oder Tiefenelektroden umfasst.

10. System nach einem der vorhergehenden Ansprüche, wobei eine Ader der implantierbaren Aderbaugruppe ein Substrat mit einer relativ flachen Geometrie, einer Breite von bis zu 3,5 mm und einer Länge von etwa 10 cm umfasst, wobei die Ader ferner mehrere elektrodenbasierte Sensoren mit einer Länge von etwa 5 mm umfasst, wobei jeder Sensor dazu konfiguriert ist, auf dem Substrat der Ader mit einem Mittenabstand von etwa 25 mm von einem benachbarten Sensor positioniert zu werden.

11. System nach einem der vorhergehenden Ansprüche, wobei das System dazu konfiguriert ist, Gehirngewebe zu stimulieren, um eine immer verfügbare Neuromodulation zu erreichen.

12. System nach einem der vorhergehenden Ansprüche, wobei das System ein oder mehrere Werkzeuge (800) beinhaltet, wobei das eine oder die mehreren Werkzeuge ein Einführwerkzeug (810) umfassen, das dazu konfiguriert ist, eine oder mehrere Adern der Aderbaugruppe unter eine Haut des Patienten zu tunneln.

13. System nach einem der vorhergehenden Ansprüche, wobei es konfiguriert ist, um selbstverabreichtes, immer verfügbares Neurofeedback bereitzustellen.

14. System nach einem der vorhergehenden Ansprüche, wobei ein oder mehrere Sensoren der implantierbaren Aderbaugruppe eine oder mehrere funktionelle Nahinfrarotspektroskopie, fNIRS, Sensoren und/oder mindestens eine Foramen-ovale-Elektrode umfassen.

## Revendications

1. Système d'interface neuronale (10) destinée à un patient comprenant :
un dispositif capteur implantable (100) comprenant :
un ensemble de fils implantable (150) destiné à être implanté au-dessus du crâne et sous la peau du patient et à enregistrer des informations sur les paramètres physiologiques du patient, et
un émetteur implantable (110) destiné à recevoir les informations de paramètres physiologiques provenant de l'ensemble de fils implantable et à transmettre des données de patient qui sont basées sur les informations de paramètres physiologiques ;
un conduit (152) qui relie l'ensemble de fils implantable à l'émetteur implantable, le conduit comprenant un fil central (154) ; et
un dispositif de traitement externe (200) destiné à recevoir les données du patient provenant de l'émetteur implantable,
ledit ensemble de fils implantable comprenant de multiples électrodes (160) et de multiples fils souples (151) s'étendant depuis le conduit suivant des angles compris entre 5 et 45°, dans un agencement pliable en quinconce à partir de côtés alternés de l'axe central du conduit avec une distance de séparation axiale d'au moins 5 mm les uns des autres, chaque fil comprenant au moins une électrode et présentant une longueur de 5 cm à 15 cm.

2. Système selon la revendication 1, lesdits fils étant conçus pour être tunnelisés sous la peau.

3. Système selon l'une quelconque des revendications 1 ou 2, lesdites électrodes comprenant deux électrodes à facettes ou plus qui s'étendent sur moins de 180 degrés d'un segment circonférentiel, lesdites deux électrodes à facettes ou plus pouvant être sélectionnées individuellement et lesdites deux électrodes à facettes ou plus comprenant une première électrode à facettes destinée à enregistrer les paramètres physiologiques et une seconde électrode à facettes utilisée pour la suppression du bruit, la seconde électrode à facettes étant conçue pour être orientée à l'opposé du crâne du patient après une implantation.

4. Système selon l'une quelconque des revendications 1 ou 2,
lesdites électrodes comprenant au moins une électrode en anneau concentrique entourant une électrode centrale, l'ensemble de fils implantable comprenant au moins une électrode comprenant une partie blindée.

5. Système selon l'une quelconque des revendications précédentes, chaque fil comprenant entre trois et dix électrodes, les multiples fils étant conçus pour être pliés en une géométrie de tube unique.

6. Système selon l'une quelconque des revendications précédentes, au moins une partie du dispositif capteur implantable étant biodégradable, ladite partie biodégradable comprenant au moins une partie d'un composant choisi dans le groupe constitué par : un fil ou un autre conduit de l'ensemble de fils implantable ; une électrode du fil implantable ; une tige du fil implantable ; un élément de stimulation ; et des combinaisons de ceux-ci.

7. Système selon l'une quelconque des revendications précédentes, chaque fil comprenant un ou plusieurs éléments de renforcement axiaux, chaque élément de renforcement axial comprenant un filament de renforcement, ledit filament de renforcement comprenant des matériaux et une construction conçue pour résister aux forces subies lors de la mise en prise avec un ou plusieurs outils de tunnelisation et/ou lors de la mise en prise avec un ou plusieurs outils de retrait.

8. Système selon l'une quelconque des revendications précédentes, ledit ensemble de fils implantable comprenant au moins un capteur, ledit système comprenant en outre un marqueur visualisable positionné par rapport audit au moins un capteur, ledit marqueur visualisable comprenant une diode infrarouge ou un marqueur choisi dans le groupe constitué par : un marqueur radio-opaque ; un marqueur ultrasonore ; un marqueur magnétique ; et des combinaisons de ceux-ci.

9. Système selon l'une quelconque des revendications précédentes, ledit système comprenant en outre au moins un capteur intracrânien relié de manière fonctionnelle à l'émetteur implantable, ledit au moins un capteur intracrânien comprenant des électrodes épidurales, sous-durales et/ou profondes.

10. Système selon l'une quelconque des revendications précédentes, un fil de l'ensemble de fils implantable comprenant un substrat doté d'une géométrie relativement plate, la largeur faisant jusqu'à 3,5 mm et la longueur faisant environ 10 cm, ledit fil comprenant en outre de multiples capteurs à base d'électrodes dotés d'une longueur d'environ 5 mm, chaque capteur étant conçu pour être positionné sur le substrat du fil avec une distance de séparation centre à centre d'environ 25 mm par rapport à un capteur voisin.

11. Système selon l'une quelconque des revendications précédentes, ledit système étant conçu pour stimuler le tissu cérébral de manière à réaliser une neuromodulation toujours disponible.

12. Système selon l'une quelconque des revendications précédentes, ledit système comprenant un ou plusieurs outils (800), lesdits un ou plusieurs outils comprenant un outil d'introduction (810) conçu pour tunneliser un ou plusieurs fils de l'ensemble de fils sous la peau du patient.

13. Système selon l'une quelconque des revendications précédentes, lequel est conçu pour fournir un neurofeedback auto-administré toujours disponible.

14. Système selon l'une quelconque des revendications précédentes, un ou plusieurs capteurs de l'ensemble de fils implantable comprenant un ou plusieurs capteurs fonctionnels de spectroscopie dans l'infrarouge proche, fNIRS, et/ou au moins une électrode ovale pour foramen.
